# EUROPEAN PATENT SPECIFICATION

(11) **EP 4 480 483 B1**
(45) Date of publication and mention of the grant of the patent: **28.10.2026**
(21) Application number: 24204028.5
(22) Date of filing: 22.07.2021
(51) Int. Cl.: C07D 403/12, A61P 17/00, A61K 31/517

(54) **SOLID STATE FORMS OF BELUMOSUDIL AND BELUMOSUDIL SALTS**
FESTE FORMEN VON BLUMOSUDIL UND BLUMOSUDILSALZEN
FORMES À L'ÉTAT SOLIDE DE SELS DE BÉLUNOSUDIL ET DE BÉLUNOSUDIL

(30) Priority: 22.07.2020 US 202063054918 P; 08.09.2020 US 202063075368 P; 22.10.2020 US 202063104329 P; 14.01.2021 US 202163137212 P
(43) Date of publication of application: 25.12.2024
(62) Document number(s) of the earlier application(s) in accordance with Art. 76 EPC: 21755332.0 / 4 185 580
(73) Proprietor: Assia Chemical Industries Ltd., Tel Aviv 6944020 (IL)
(72) Inventor: LAPIDO, Polina, Lezion (IL); SHUMACHER, Inbal, Kadima (IL); SHAUL, Ofir, Hasharon (IL)
(74) Representative: D Young & Co LLP

(56) References cited:
- EP-A1- 3 875 078
- WO-A2-2006/105081
- MINO R CAIRA ED - MONTCHAMP JEAN-LUC: "Crystalline Polymorphism of Organic Compounds", TOPICS IN CURRENT CHEMISTRY; [TOPICS IN CURRENT CHEMISTRY], SPRINGER, BERLIN, DE, vol. 198, 1 January 1998 (1998-01-01), pages 163 - 208, XP008166276, ISSN: 0340-1022, [retrieved on 19990226], DOI: 10.1007/3-540-69178-2_5

## Description

### FIELD OF THE DISCLOSURE

The present disclosure encompasses a solid state form of Belumosudil mesylate (form M3), processes for preparation thereof, and pharmaceutical compositions thereof.

### BACKGROUND OF THE DISCLOSURE

Belumosudil, 2-(3-(4-((1H-Indazol-5-yl)amino)quinazolin-2-yl)phenoxy)-N-isopropylacetamide, has the following chemical structure:

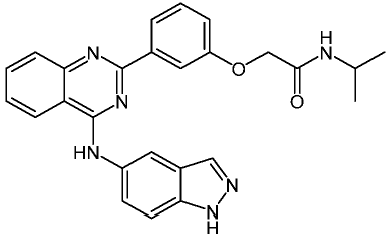

Belumosudil is a ROCK2 inhibitor, and it is developed for the treatment of Graft-Versus-Host Disease including Chronic Graft-Versus-Host Disease, Systemic Sclerosis including Diffuse Cutaneous Systemic Sclerosis, Fibrosis including Idiopathic Pulmonary Fibrosis, Plaque Psoriasis, and Systemic Scleroderma.

The compound is described in International Publication No. WO 2006/105081. Crystalline forms of Belumosudil are disclosed in International Publication No. WO 2021/129589.

Polymorphism, the occurrence of different crystalline forms, is a property of some molecules and molecular complexes. A single molecule may produce a rise to a variety of polymorphs having distinct crystal structures and physical properties like melting point, thermal behaviors (e.g., measured by thermogravimetric analysis ("TGA"), or differential scanning calorimetry ("DSC")), X-ray diffraction (XRD) pattern, infrared absorption fingerprint, and solid state (¹³C) NMR spectrum. One or more of these techniques may be used to distinguish different polymorphic forms of a compound.

Different salts and solid state forms (including solvated forms) of an active pharmaceutical ingredient may possess different properties. Such variations in the properties of different salts and solid state forms and solvates may provide a basis for improving formulation, for example, by facilitating better processing or handling characteristics, changing the dissolution profile in a favorable direction, or improving stability (polymorph as well as chemical stability) and shelf-life. These variations in the properties of different salts and solid state forms may also offer improvements to the final dosage form, for instance, if they serve to improve bioavailability. Different salts and solid state forms and solvates of an active pharmaceutical ingredient may also produce a rise to a variety of polymorphs or crystalline forms, which may in turn provide additional opportunities to assess variations in the properties and characteristics of a solid active pharmaceutical ingredient.

Discovering new solid state forms and solvates of a pharmaceutical product may yield materials having desirable processing properties, such as ease of handling, ease of processing, storage stability, and ease of purification or as desirable intermediate crystal forms that facilitate conversion to other polymorphic forms. New solid state forms of a pharmaceutically useful compound can also provide an opportunity to improve the performance characteristics of a pharmaceutical product. It enlarges the repertoire of materials that a formulation scientist has available for formulation optimization, for example by providing a product with different properties, including a different crystal habit, higher crystallinity, or polymorphic stability, which may offer better processing or handling characteristics, improved dissolution profile, or improved shelf-life (chemical/physical stability). For at least these reasons, there is a need for additional solid state forms (including solvated forms) of Belumosudil salts.

### SUMMARY OF THE DISCLOSURE

The present disclosure provides crystalline polymorph Form M3 of Belumosudil Mesylate, processes for preparation thereof, and pharmaceutical compositions thereof. These crystalline polymorphs can be used to prepare other solid state forms of Belumosudil, Belumosudil salts and their solid state forms.

The present disclosure also provides uses of the said solid state form M3 of Belumosudil Mesylate in the preparation of other solid state forms of Belumosudil or salts thereof.

The present disclosure provides crystalline polymorph Form M3 of Belumosudil Mesylate for use in medicine, including for the treatment of Graft-Versus-Host Disease including Chronic Graft-Versus-Host Disease, Systemic Sclerosis including Diffuse Cutaneous Systemic Sclerosis, Fibrosis including Idiopathic Pulmonary Fibrosis, Plaque Psoriasis, Systemic Scleroderma. Particularly, the present disclosure provides crystalline polymorph Form M3 of Belumosudil Mesylate for use in medicine, including for the treatment of Chronic Graft-Versus-Host Disease and/or Systemic Sclerosis.

The present disclosure also encompasses the use of crystalline polymorph Form M3 of Belumosudil Mesylate of the present disclosure for the preparation of pharmaceutical compositions and/or formulations.

In another aspect, the present disclosure provides pharmaceutical compositions comprising crystalline polymorph Form M3 of Belumosudil Mesylate according to the present disclosure.

The present disclosure includes processes for preparing the above mentioned pharmaceutical compositions. The processes include combining crystalline polymorph Form M3 of Belumosudil Mesylate with at least one pharmaceutically acceptable excipient.

The crystalline polymorph Form M3 of Belumosudil Mesylate as defined herein and the pharmaceutical compositions or formulations of the crystalline polymorph Form M3 Belumosudil Mesylate may be used as medicaments, such as for the treatment of Graft-Versus-Host Disease including Chronic Graft-Versus-Host Disease, Systemic Sclerosis including Diffuse Cutaneous Systemic Sclerosis, Fibrosis including Idiopathic Pulmonary Fibrosis, Plaque Psoriasis, Systemic Scleroderma. Particularly, the crystalline polymorph Form M3 of Belumosudil Mesylate as defined herein and the pharmaceutical compositions or formulations of the crystalline polymorph Form M3 of Belumosudil Mesylate may be used as medicaments, for the treatment of Chronic Graft-Versus-Host Disease and/or Systemic Sclerosis.

The present disclosure also provides uses of crystalline polymorph Form M3 of Belumosudil Mesylate of the present disclosure, or at least one of the above pharmaceutical compositions, for the manufacture of medicaments for treating e.g., Graft-Versus-Host Disease including Chronic Graft-Versus-Host Disease, Systemic Sclerosis including Diffuse Cutaneous Systemic Sclerosis, Fibrosis including Idiopathic Pulmonary Fibrosis, Plaque Psoriasis, Systemic Scleroderma, and particularly for treating Chronic Graft-Versus-Host Disease and/or Systemic Sclerosis.

### BRIEF DESCRIPTION OF THE DRAWINGS

Figure 1 shows an X-ray powder diffraction pattern (XRPD) of amorphous Belumosudil.
Figure 2 shows a characteristic X-ray powder diffraction pattern (XRPD) of Belumosudil Form B1.
Figure 3 shows a characteristic X-ray powder diffraction pattern (XRPD) of Belumosudil Form B2.
Figure 4 shows a characteristic X-ray powder diffraction pattern (XRPD) of Belumosudil Form B3.
Figure 5 shows a characteristic X-ray powder diffraction pattern (XRPD) of Belumosudil Mesylate Form M1.
Figure 6 shows a characteristic X-ray powder diffraction pattern (XRPD) of Belumosudil form B4.
Figure 7 shows a characteristic X-ray powder diffraction pattern (XRPD) of Belumosudil Mesylate form M2.
Figure 8 shows a characteristic X-ray powder diffraction pattern (XRPD) of Belumosudil Mesylate form M3.
Figure 9 shows a characteristic X-ray powder diffraction pattern (XRPD) of Belumosudil form B5.
Figure 10 shows a characteristic X-ray powder diffraction pattern (XRPD) of amorphous Belumosudil Mesylate.
Figure 11 shows a characteristic X-ray powder diffraction pattern (XRPD) of Belumosudil Mesylate form M4.
Figure 12 shows a characteristic X-ray powder diffraction pattern (XRPD) of Belumosudil Mesylate form M5.
Figure 13 shows a characteristic X-ray powder diffraction pattern (XRPD) of Belumosudil Besylate form BS1.
Figure 14 shows a characteristic X-ray powder diffraction pattern (XRPD) of Belumosudil Tosylate form T1.
Figure 15 shows a characteristic X-ray powder diffraction pattern (XRPD) of Belumosudil Tosylate form T2.
Figure 16 shows a characteristic solid state ¹³C NMR spectrum of Form M1 of Belumosudil Mesylate (full range 200-0 ppm).
Figure 17 shows a characteristic solid state ¹³C NMR spectrum of Form M1 of Belumosudil Mesylate 200-100 ppm).
Figure 18 shows a characteristic solid state ¹³C NMR spectrum of Form M1 of Belumosudil Mesylate (100-0 ppm).
Figure 19 shows a characteristic solid state ¹³C NMR spectrum of Form M2 of Belumosudil Mesylate (full range 200-0 ppm).
Figure 20 shows a characteristic solid state ¹³C NMR spectrum of Form M2 of Belumosudil Mesylate 200-100 ppm).
Figure 21 shows a characteristic solid state ¹³C NMR spectrum of Form M2 of Belumosudil Mesylate (100-0 ppm).
Figure 22 shows a characteristic solid state ¹³C NMR spectrum of Form BS1 of Belumosudil Besylate (full range 200-0 ppm).
Figure 23 shows a characteristic solid state ¹³C NMR spectrum of Form BS1 of Belumosudil Besylate 200-100 ppm).
Figure 24 shows a characteristic solid state ¹³C NMR spectrum of Form BS1 of Belumosudil Besylate (100-0 ppm).

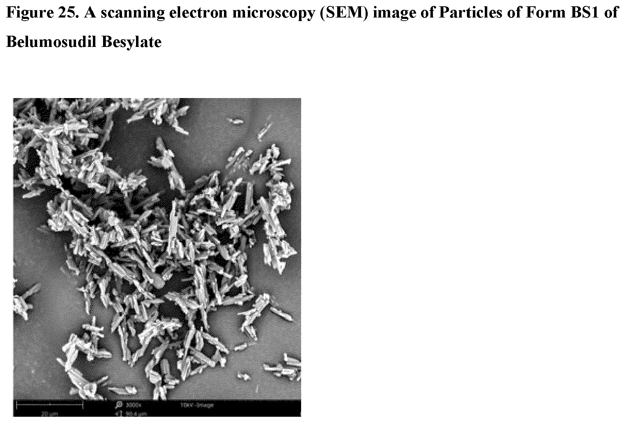

Figure 25 shows a scanning electron microscopy (SEM) image of particles of Form BS1 of Belumosudil Besylate.
Figure 26 shows a characteristic solid state ¹³C NMR spectrum of Form T1 of Belumosudil Tosylate (full range 200-0 ppm).
Figure 27 shows a characteristic solid state ¹³C NMR spectrum of Form T1 of Belumosudil Tosylate 200-100 ppm).
Figure 28 shows a characteristic solid state ¹³C NMR spectrum of Form T1 of Belumosudil Tosylate (100-0 ppm).

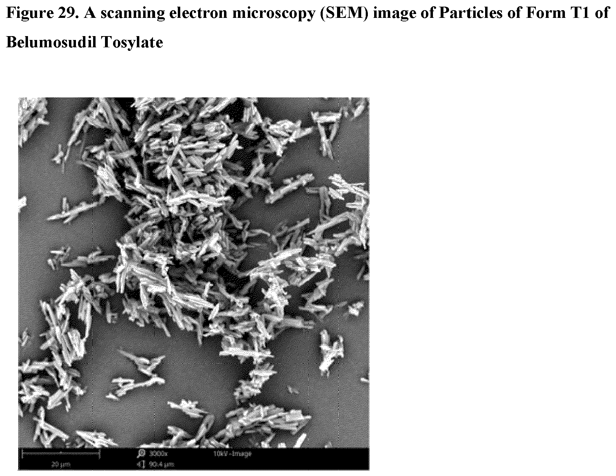

Figure 29 shows a scanning electron microscopy (SEM) image of particles of Form T1 of Belumosudil Tosylate.
Figure 30 shows a characteristic solid state ¹³C NMR spectrum of Form T2 of Belumosudil Tosylate (full range 200-0 ppm).
Figure 31 shows a characteristic solid state ¹³C NMR spectrum of Form T2 of Belumosudil Tosylate 200-100 ppm).
Figure 32 shows a characteristic solid state ¹³C NMR spectrum of Form T2 of Belumosudil Tosylate (100-0 ppm).

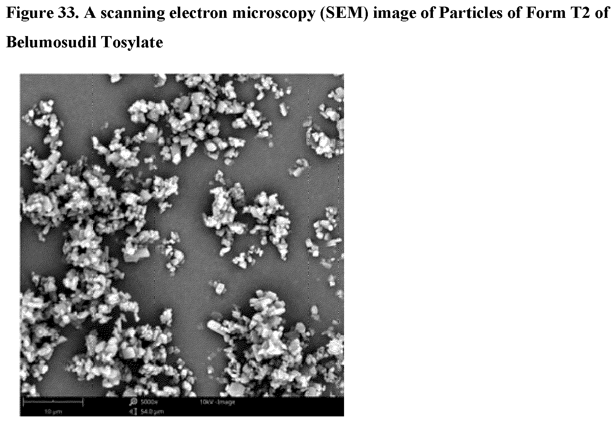

Figure 33 shows a scanning electron microscopy (SEM) image of particles of Form T2 of Belumosudil Tosylate.

### DETAILED DESCRIPTION OF THE DISCLOSURE

The present disclosure encompasses solid state form M3 of Belumosudil Mesylate, processes for preparation thereof, and pharmaceutical compositions thereof.

Solid state properties of Belumosudil, Belumosudil salts, and crystalline polymorphs thereof can be influenced by controlling the conditions under which Belumosudil and Belumosudil salts, and crystalline polymorphs thereof are obtained in solid form.

A solid state form (or polymorph) may be referred to herein as polymorphically pure or as substantially free of any other solid state (or polymorphic) forms. As used herein in this context, the expression "substantially free of any other forms" will be understood to mean that the solid state form contains about 20% (w/w) or less, about 10% (w/w) or less, about 5% (w/w) or less, about 2% (w/w) or less, about 1% (w/w) or less, or about 0% of any other forms of the subject compound as measured, for example, by XRPD. Thus, a crystalline polymorph of Belumosudil or Belumosudil salt, described herein as substantially free of any other solid state forms would be understood to contain greater than about 80% (w/w), greater than about 90% (w/w), greater than about 95% (w/w), greater than about 98% (w/w), greater than about 99% (w/w), or about 100% of the subject crystalline polymorph of Belumosudil or Belumosudil salt. In some embodiments of the disclosure, the described crystalline polymorph Form M3 of Belumosudil Mesylate, may contain from about 1% to about 20% (w/w), from about 5% to about 20% (w/w), or from about 5% to about 10% (w/w) of one or more other crystalline polymorph of Belumosudil Mesylate described herein as substantially free of any other solid state forms would be understood to contain greater than about 80% (w/w), greater than about 90% (w/w), greater than about 95% (w/w), greater than about 98% (w/w), greater than about 99% (w/w), or about 100% of the subject crystalline polymorph of Belumosudil Mesylate. In some embodiments of the disclosure, the described crystalline polymorph of Belumosudil Mesylate may contain from about 1% to about 20% (w/w), from about 5% to about 20% (w/w), or from about 5% to about 10% (w/w) of one or more other crystalline polymorph of Belumosudil Mesylate.

Depending on which other crystalline polymorphs a comparison is made, the crystalline polymorph Form M3 of Belumosudil Mesylate of the present disclosure may have advantageous properties selected from at least one of the following: chemical purity, flowability, solubility, dissolution rate, morphology or crystal habit, stability, such as chemical stability as well as thermal and mechanical stability with respect to polymorphic conversion, stability towards dehydration and/or storage stability, low content of residual solvent, a lower degree of hygroscopicity, flowability, and advantageous processing and handling characteristics such as compressibility and bulk density.

A solid state form, such as a crystal form or an amorphous form, may be referred to herein as being characterized by graphical data "as depicted in" or "as substantially depicted in" a Figure. Such data include, for example, powder X-ray diffractograms and solid state NMR spectra. As is well-known in the art, the graphical data potentially provides additional technical information to further define the respective solid state form (a so-called "fingerprint") which cannot necessarily be described by reference to numerical values or peak positions alone. In any event, the skilled person will understand that such graphical representations of data may be subject to small variations, e.g., in peak relative intensities and peak positions due to certain factors such as, but not limited to, variations in instrument response and variations in sample concentration and purity, which are well known to the skilled person. Nonetheless, the skilled person would readily be capable of comparing the graphical data in the Figures herein with graphical data generated for an unknown crystal form and confirm whether the two sets of graphical data are characterizing the same crystal form or two different crystal forms. A crystal form of Belumosudil or Belumosudil salt, referred to herein as being characterized by graphical data "as depicted in" or "as substantially depicted in" a Figure will thus be understood to include any crystal forms of Belumosudil or Belumosudil salt, characterized with the graphical data having such small variations, as are well known to the skilled person, in comparison with the Figure.

As used herein, and unless stated otherwise, the term "anhydrous" in relation to crystalline forms of Belumosudil, Belumosudil Mesylate, Belumosudil Tosylate and/or Belumosudil Besylate, relates to a crystalline form of Belumosudil or Belumosudil salt which does not include any crystalline water (or other solvents) in a defined, stoichiometric amount within the crystal. Moreover, an "anhydrous" form would generally not contain more than 1% (w/w), of either water or organic solvents as measured for example by TGA.

The term "solvate," as used herein and unless indicated otherwise, refers to a crystal form that incorporates a solvent in the crystal structure. When the solvent is water, the solvate is often referred to as a "hydrate." The solvent in a solvate may be present in either a stoichiometric or in a non-stoichiometric amount.

As used herein, the term "isolated" in reference to crystalline polymorph of Belumosudil, or Belumosudil salt (e.g., Belumosudil Mesylate, Belumosudil Tosylate and/or Belumosudil Besylate) of the present disclosure corresponds to a crystalline polymorph of Belumosudil or Belumosudil salt, that is physically separated from the reaction mixture in which it is formed.

As used herein, unless stated otherwise, the XRPD measurements are taken using copper Kα radiation wavelength 1.5418 Å. XRPD peaks reported herein are measured using CuK α radiation, λ = 1.5418 Å, typically at a temperature of 25 ± 3°C.

As used herein, ¹³C NMR spectra are preferably measured at 125 MHz at magic angle spinning (MAS) frequency ωr/2π=11 kHz

As used herein, unless stated otherwise, TGA analysis is preferably carried out at a heating rate of 10 °C/min to 250 °C, preferably with a nitrogen flow of 40 ml/min.

A thing, *e.g.,* a reaction mixture, may be characterized herein as being at, or allowed to come to "room temperature" or "ambient temperature", often abbreviated as "RT." This means that the temperature of the thing is close to, or the same as, that of the space, *e.g.,* the room or fume hood, in which the thing is located. Typically, room temperature is from about 20°C to about 30°C, or about 22°C to about 27°C, or about 25°C.

The amount of solvent employed in a chemical process, e.g., a reaction or crystallization, may be referred to herein as a number of "volumes" or "vol" or "V." For example, a material may be referred to as being suspended in 10 volumes (or 10 vol or 10V) of a solvent. In this context, this expression would be understood to mean milliliters of the solvent per gram of the material being suspended, such that suspending a 5 grams of a material in 10 volumes of a solvent means that the solvent is used in an amount of 10 milliliters of the solvent per gram of the material that is being suspended or, in this example, 50 mL of the solvent. In another context, the term "v/v" may be used to indicate the number of volumes of a solvent that are added to a liquid mixture based on the volume of that mixture. For example, adding solvent X (1.5 v/v) to a 100 ml reaction mixture would indicate that 150 mL of solvent X was added.

A process or step may be referred to herein as being carried out "overnight." This refers to a time interval, e.g., for the process or step, that spans the time during the night, when that process or step may not be actively observed. This time interval is from about 8 to about 20 hours, or about 10-18 hours, in some cases about 16 hours.

As used herein, the term "reduced pressure" refers to a pressure that is less than atmospheric pressure. For example, reduced pressure is about 10 mbar to about 50 mbar.

As used herein and unless indicated otherwise, the term "ambient conditions" refer to atmospheric pressure and a temperature of 22-24°C.

Only crystalline form M3 of Belumosudil mesylate and subject-matter related thereto as included in the claims is part of the invention.

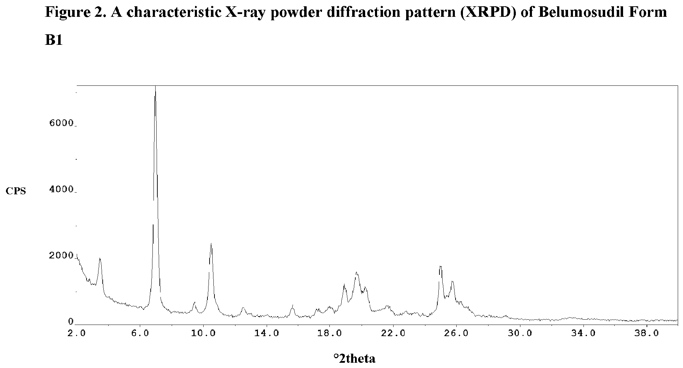

Disclosed is a crystalline polymorph of Belumosudil, designated form B1. The crystalline Form B1 of Belumosudil may be characterized by data selected from one or more of the following: an X-ray powder diffraction pattern substantially as depicted in Figure 2; an X-ray powder diffraction pattern having peaks at 3.5, 6.9, 10.5, 24.9 and 25.7 degrees 2-theta ± 0.2 degrees 2-theta; and combinations of these data.

Crystalline Form B1 of Belumosudil may be further characterized by an X-ray powder diffraction pattern having peaks at 3.5, 6.9, 10.5, 24.9 and 25.7 degrees 2-theta ± 0.2 degrees 2-theta, and also having any one, two, three, four or five additional peaks selected from 9.4, 12.5, 15.7, 18.9 and 19.7 degrees 2-theta ± 0.2 degrees 2-theta.

Crystalline Form B1 of Belumosudil may be alternatively characterized by an X-ray powder diffraction pattern having peaks at 3.5, 6.9, 9.4, 10.5, 12.5, 15.7, 18.9, 19.7, 24.9, and 25.7 degrees 2-theta ± 0.2 degrees 2-theta.

Crystalline Form B1 of Belumosudil may be isolated.

Crystalline Form B1 of Belumosudil may be characterized by each of the above characteristics alone/or by all possible combinations, e.g., an XRPD pattern having peaks at 3.5, 6.9, 10.5, 24.9 and 25.7 degrees 2-theta ± 0.2 degrees 2-theta; an XRPD pattern as depicted in Figure 2, and combinations thereof.

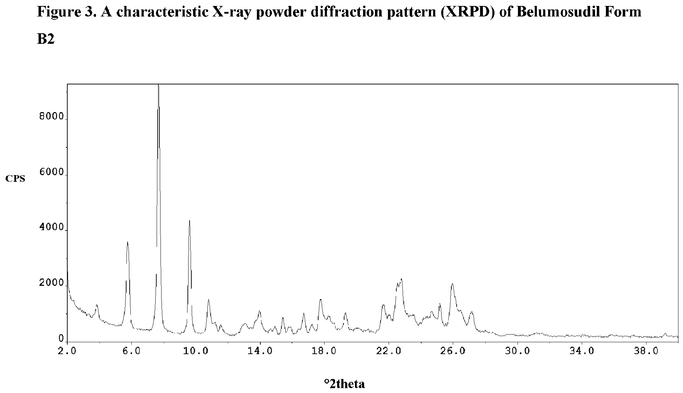

Disclosed is a crystalline polymorph of Belumosudil, designated form B2. The crystalline Form B2 of Belumosudil may be characterized by data selected from one or more of the following: an X-ray powder diffraction pattern substantially as depicted in Figure 3; an X-ray powder diffraction pattern having peaks at 3.8, 5.7, 7.6, 9.6 and 25.9 degrees 2-theta ± 0.2 degrees 2-theta; and combinations of these data.

Crystalline Form B2 of Belumosudil may be further characterized by an X-ray powder diffraction pattern having peaks at 3.8, 5.7, 7.6, 9.6 and 25.9 degrees 2-theta ± 0.2 degrees 2-theta, and also having any one, two, three, four or five additional peaks selected from 10.7, 15.4, 16.6, 17.8 and 19.3 degrees 2-theta ± 0.2 degrees 2-theta.

Crystalline Form B2 of Belumosudil may be alternatively characterized by an X-ray powder diffraction pattern having peaks at 3.8, 5.7, 7.6, 9.6, 10.7, 15.4, 16.6, 17.8, 19.3 and 25.9 degrees 2-theta ± 0.2 degrees 2-theta.

Crystalline Form B2 of Belumosudil may be isolated.

Crystalline Form B2 of Belumosudil may be characterized by each of the above characteristics alone/or by all possible combinations, e.g., an XRPD pattern having peaks at 3.8, 5.7, 7.6, 9.6 and 25.9 degrees 2-theta ± 0.2 degrees 2-theta; an XRPD pattern as depicted in Figure 3, and combinations thereof.

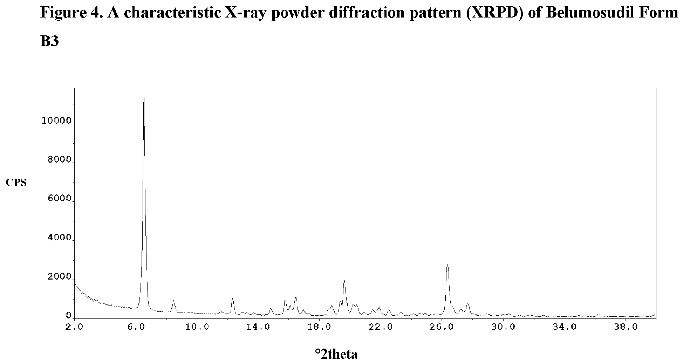

Disclosed is a crystalline polymorph of Belumosudil, designated form B3. The crystalline Form B3 of Belumosudil may be characterized by data selected from one or more of the following: an X-ray powder diffraction pattern substantially as depicted in Figure 4; an X-ray powder diffraction pattern having peaks at 6.5, 8.4, 12.2, 19.6 and 26.3 degrees 2-theta ± 0.2 degrees 2-theta; and combinations of these data.

Crystalline Form B3 of Belumosudil may be further characterized by an X-ray powder diffraction pattern having peaks at 6.5, 8.4, 12.2, 19.6 and 26.3 degrees 2-theta ± 0.2 degrees 2-theta, and also having any one, two, three, four or five additional peaks selected from 14.7, 15.4, 16.4, 18.8 and 22.5 degrees 2-theta ± 0.2 degrees 2-theta.

Crystalline Form B3 of Belumosudil may be alternatively characterized by an X-ray powder diffraction pattern having peaks at 6.5, 8.4, 12.2, 14.7, 15.4, 16.4, 18.8, 19.6, 22.5, and 26.3 degrees 2-theta ± 0.2 degrees 2-theta.

Crystalline Form B3 of Belumosudil may be isolated.

Crystalline Form B3 of Belumosudil may be characterized by each of the above characteristics alone/or by all possible combinations, e.g., an XRPD pattern having peaks at 6.5, 8.4, 12.2, 19.6 and 26.3 degrees 2-theta ± 0.2 degrees 2-theta; an XRPD pattern as depicted in Figure 4, and combinations thereof.

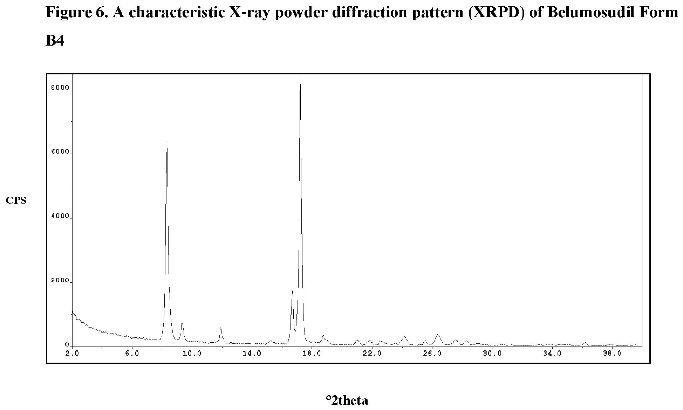

Disclosed is a crystalline polymorph of Belumosudil, designated form B4. The crystalline Form B4 of Belumosudil may be characterized by data selected from one or more of the following: an X-ray powder diffraction pattern substantially as depicted in Figure 6; an X-ray powder diffraction pattern having peaks at 8.3, 9.3, 11.9, 16.7 and 17.2 degrees 2-theta ± 0.2 degrees 2-theta; and combinations of these data.

Crystalline Form B4 of Belumosudil may be further characterized by an X-ray powder diffraction pattern having peaks at 8.3, 9.3, 11.9, 16.7 and 17.2 degrees 2-theta ± 0.2 degrees 2-theta, and also having any one, two, three, four or five additional peaks selected from 15.2, 18.7, 24.1, 25.6 and 26.2 degrees 2-theta ± 0.2 degrees 2-theta.

Crystalline Form B4 of Belumosudil may be further characterized by an X-ray powder diffraction pattern having peaks at 8.3, 9.3, 11.9, 16.7 and 17.2 degrees 2-theta ± 0.2 degrees 2-theta, and also having any one, two, three, four or five additional peaks selected from 15.2, 18.7, 24.1, 25.6 and 26.2 degrees 2-theta ± 0.2 degrees 2-theta.

Crystalline Form B4 of Belumosudil may be alternatively characterized by an X-ray powder diffraction pattern having peaks at 8.3, 9.3, 11.9, 15.2, 16.7, 17.2, 18.7, 24.1, 25.6, and 26.2 degrees 2-theta ± 0.2 degrees 2-theta.

Crystalline Form B4 of Belumosudil may be isolated.

Crystalline Form B4 of Belumosudil may be characterized by each of the above characteristics alone/or by all possible combinations, e.g., an XRPD pattern having peaks at 8.3, 9.3, 11.9, 16.7 and 17.2 degrees 2-theta ± 0.2 degrees 2-theta; an XRPD pattern as depicted in Figure 6, and combinations thereof.

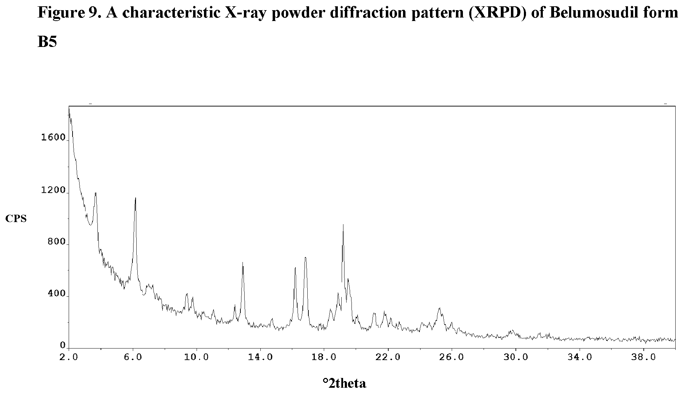

Disclosed is a crystalline polymorph of Belumosudil, designated form B5. The crystalline Form B5 of Belumosudil may be characterized by data selected from one or more of the following: an X-ray powder diffraction pattern substantially as depicted in Figure 9; an X-ray powder diffraction pattern having peaks at 6.1, 12.9, 16.2, 16.8 and 19.2 degrees 2-theta ± 0.2 degrees 2-theta; and combinations of these data.

Crystalline Form B5 of Belumosudil may be further characterized by an X-ray powder diffraction pattern having peaks at 6.1, 12.9, 16.2, 16.8 and 19.2 degrees 2-theta ± 0.2 degrees 2-theta, and also having any one, two, three, four or five additional peaks selected from 3.7, 9.4, 9.8, 12.4 and 18.4 degrees 2-theta ± 0.2 degrees 2-theta.

Crystalline Form B5 of Belumosudil may be alternatively characterized by an X-ray powder diffraction pattern having peaks at 3.7, 6.1, 9.4, 9.8, 12.4, 12.9, 16.2, 16.8, 18.4, and 19.2 degrees 2-theta ± 0.2 degrees 2-theta.

Crystalline Form B5 of Belumosudil may be isolated.

Crystalline Form B5 of Belumosudil may be characterized by each of the above characteristics alone/or by all possible combinations, e.g., an XRPD pattern having peaks at 6.1, 12.9, 16.2, 16.8 and 19.2 degrees 2-theta ± 0.2 degrees 2-theta; an XRPD pattern as depicted in Figure 9, and combinations thereof.

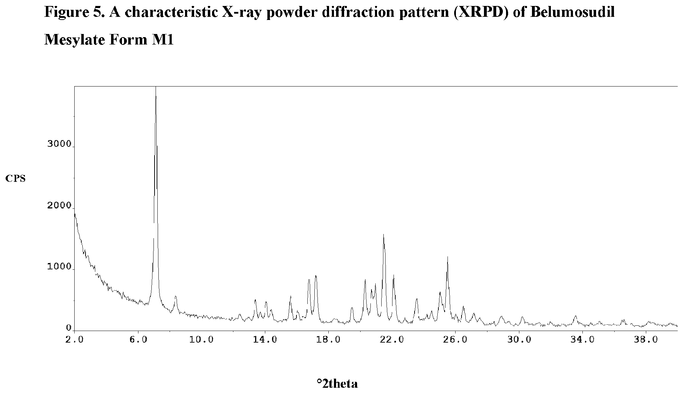

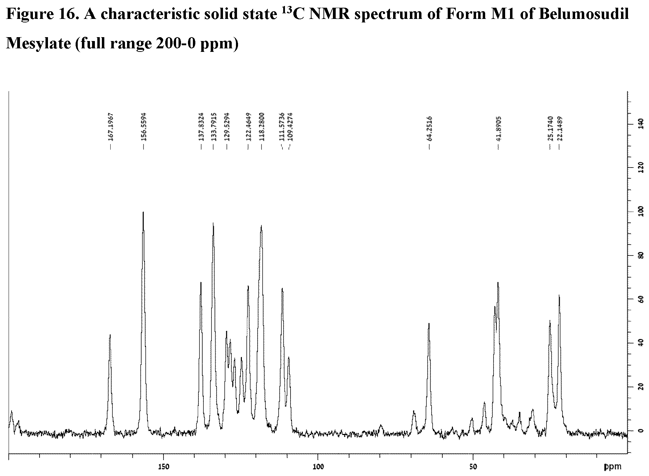

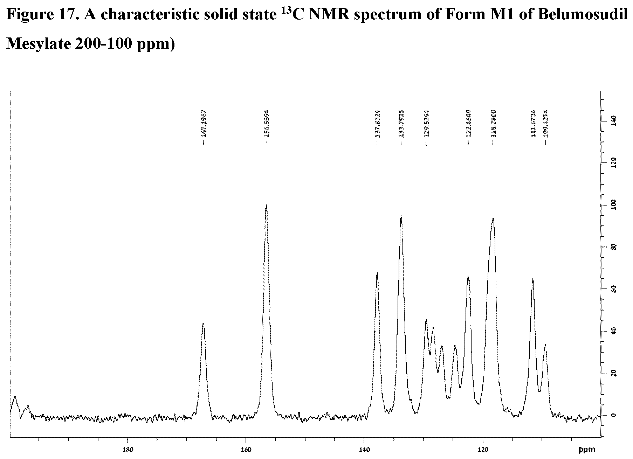

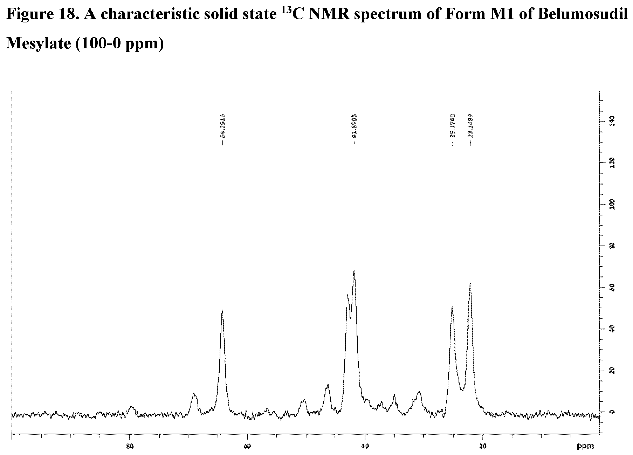

Disclosed is a crystalline polymorph of Belumosudil Mesylate, designated form M1. The crystalline Form M1 of Belumosudil Mesylate may be characterized by data selected from one or more of the following: an X-ray powder diffraction pattern substantially as depicted in Figure 5; an X-ray powder diffraction pattern having peaks at 7.1, 17.2, 20.3, 21.5 and 25.5 degrees 2-theta ± 0.2 degrees 2-theta; a solid state ¹³C NMR spectrum with characteristic peaks at 137.8, 133.8, 122.5, 118.3 and 111.6 ppm ± 0.2 ppm; a solid state ¹³C NMR spectrum having the following chemical shift absolute differences from reference peak at 167.2 ppm ± 1 ppm: 29.4, 33.4, 44.7, 48.9 and 55.6 ppm ± 0.1 ppm; a solid state ¹³C NMR spectrum substantially as depicted in any of Figures 16, 17 and 18; and combinations of these data.

Crystalline Form M1 of Belumosudil Mesylate may be further characterized by an X-ray powder diffraction pattern having peaks at 7.1, 17.2, 20.3, 21.5 and 25.5 degrees 2-theta ± 0.2 degrees 2-theta, and also having any one, two, three, four or five additional peaks selected from 8.4, 15.5, 16.8, 19.5 and 22.1 degrees 2-theta ± 0.2 degrees 2-theta.

Crystalline form M1 of Belumosudil Mesylate may be alternatively characterized by X-ray powder diffraction pattern having peaks at 7.1, 8.4, 15.5, 16.8, 17.2, 19.5, 20.3, 21.5, 22.1 and 25.5 degrees 2-theta ± 0.2 degrees 2-theta.

Crystalline form M1 of Belumosudil Mesylate may be an anhydrous form, as can be determined by TGA. Crystalline form M1 of Belumosudil Mesylate may contain 0.5% w/w or less, or 0.2% w/w or less, or 0.1 wt% or less, of total residual solvent. The residual solvent may be one or more polar solvents, preferably water, alcohols (particularly C₁₋₄ alcohols, especially methanol, ethanol, isopropanol, 1-propanol, or n-butanol) or halogenated solvents (particularly 2,2,2-trifluoroethanol or dichloromethane), or DMSO, or mixtures thereof. Particularly, crystalline form M1 of Belumosudil Mesylate may contain 0.5% w/w or less, or 0.2% w/w or less, or 0.1 wt% or less, of total residual solvent, wherein the residual solvent is: ethanol, water, or DMSO, or a combination of ethanol, water and DMSO and water, or a combination of ethanol and DMSO. Alternatively, crystalline form M1 of Belumosudil Mesylate may contain 0.5% w/w or less, or 0.2% w/w or less, or 0.1 wt% or less, of ethanol, or 0.5% w/w or less, or 0.2 w/w or less, or 0.1 wt% or less, of a mixture of ethanol and DMSO.

Crystalline form M1 of Belumosudil Mesylate may be isolated.

Crystalline Form M1 of Belumosudil Mesylate may be non-hygroscopic. Particularly, Form M1 of Belumosudil Mesylate may be polymorphically stable at up to 100% relative humidity at room temperature for at least 7 days.

Crystalline Form M1 of Belumosudil Mesylate may be characterized by each of the above characteristics alone/or by all possible combinations, e.g., an XRPD pattern having peaks at 7.1, 17.2, 20.3, 21.5 and 25.5 degrees 2-theta ± 0.2 degrees 2-theta; an XRPD pattern as depicted in Figure 5, and combinations thereof.

Crystalline form M1 of Belumosudil Mesylate may be prepared by crystallisation from a mixture comprising Belumosudil Mesylate and one or more polar solvents, preferably a polar organic solvent, and particularly wherein the polar solvent is an alcohol or halogenated solvent, or mixtures thereof. Examples of suitable solvents include, but are not limited to, methanol, ethanol, isopropanol, 1-propanol, n-butanol, dichloromethane, and combinations thereof; and preferably ethanol, isopropanol, n-butanol or 1-propanol. Form M1 may be prepared by a process comprising:
(a) providing a mixture of Belumosudil Mesylate in at least one polar solvent, optionally with heating;
(b) optionally stirring the mixture, optionally at elevated temperature;
(c) optionally cooling the mixture, preferably to room temperature;
(d) optionally stirring the cooled mixture; and
(e) optionally isolating crystalline form M1 of Belumosudil Mesylate from the mixture.

The mixture in step (a) may be prepared by:
(i) providing a mixture of Belumosudil free base in a polar solvent;
(ii) combining the mixture with Methanesulfonic acid;
(iii) optionally heating the mixture; and
(iv) optionally adding a further polar solvent.

Preferably, the process for preparing Form M1 of Belumosudil Mesylate as described herein is carried out in the absence of water or substantially in the absence of water, particularly 2 wt% or less, 1 wt% or less, 0.5 wt% or less, 0.2 wt% or less, or 0.1 wt% or less of water.

In the process for preparing form M1 as described herein, the solvent in step (i) is preferably an organic solvent, particularly comprising an alcohol, a halogenated solvent, or mixtures thereof. Preferably the solvent is selected from the group consisting of methanol, ethanol, isopropanol, 1-propanol, n-butanol, and dichloromethane or mixtures thereof, and particularly, ethanol, isopropanol, 1-propanol, n-butanol.

In step (i), the ratio of solvent to Belumosudil may be: about 10 to about 40 ml per gram of Belumosudil, about 14 to about 35 ml per gram of Belumosudil or about 16 to about 20 to about 32 ml per gram of Belumosudil, or about 18 to about 24 ml per gram of Belumosudil, and optionally about 20 ml per gram of Belumosudil. The mixture in step (i) may be a solution or a slurry.

In step (ii) of the process for preparing form M1, combining of methanesulfonic acid to Belumosudil may be in any order. Preferably, methanesulfonic acid may be added to the mixture of Belumosudil in the solvent. The addition may be carried out portion-wise or dropwise. The methanesulfonic acid is optionally added in an amount of: about 0.7 to about 1.5 molar equivalents, about 0.9 to about 1.3 molar equivalents, about 1.0 to about 1.2 molar equivalents, or about 1.1 molar equivalents relative to Belumosudil. Preferably, step (ii) comprises adding Methanesulfonic acid to the mixture of Belumosudil and solvent.

In step (iii) of the process for preparing form M1, the mixture may be optionally heated. The heating may be to a temperature of about 30°C to about 70°C, about 40°C to about 60°C, about 45°C to about 55°C, or about 50°C.

In step (iv), a further polar solvent may be added to the reaction mixture. The further solvent may be any solvent which is a poor solvent for Belumosudil Mesylate, i.e., a solvent in which Belumosudil Mesylate has low solubility. The further polar solvent may be the same solvent as that used in step (i)) or may be different. The further solvent is preferably selected from the group consisting of methanol, ethanol, isopropanol, 1-propanol, n-butanol, and dichloromethane or mixtures thereof. Preferably, the further polar solvent in step (iv) is selected from methanol, ethanol, isopropanol, 1-propanol, and n-butanol. More preferably, the further polar solvent in step (iv) is ethanol. The further polar solvent in step (iv) can be added in any suitable amount. Preferably, the v/v ratio of the further polar solvent to the solvent in step (i) may be from: about 5 : 1 to about 1 : 5, or about 2 : 1 to about 1 : 2, or about 1.5 : 1 to about 1 : 1.5, or about 1.3 : 1 to about 1 : 1.3, and preferably about 1 : 1.3.

Alternatively, in the process for preparing Form M1, the mixture in step (a) may be prepared by:
(i-a) combining Belumosudil Mesylate with a polar solvent, optionally with heating, to form a solution; and optionally
(ii-a) adding an organic antisolvent.

The polar solvent in step (i-a) is preferably an organic solvent, more preferably selected from the group consisting of, 2,2,2-Trifluoroethanol (TFE), and DMSO or mixtures thereof. Particularly, the solvent is selected from the group consisting of TFE and DMSO. In step (i-a), the ratio of solvent to Belumosudil Mesylate may be from: about 5 to about 50 ml, about 7 to about 40 ml, about 9 to about 35 ml, or about 10V to about 30 ml per gram of Belumosudil Mesylate.

The organic antisolvent in step (ii-a) can be any organic solvent in which Belumosudil Mesylate is poorly soluble. Preferably the organic antisolvent in step (ii-a) is selected from the group consisting of acetonitrile, dioxane, methyl ethyl ketone, methyl t-butyl ketone, ethanol, isopropanol, 1-propanol, n-butanol, and more particularly, ethanol.

In the process for preparing form M1 as described herein, the mixture in step (a) may be a solution or a slurry. The mixture in step (a) is preferably in the form of a slurry. The mixture in step (a) may be at room temperature, or may be heated. The heating may be to a temperature of about 30°C to about 70°C, about 40°C to about 60°C, about 45°C to about 55°C, or about 50°C. Alternatively, the mixture may be at room temperature.

In the process for preparing Form M1 as described herein, step (b) is carried out, preferably by stirring at a temperature of: about 30°C to about 70°C, about 40°C to about 60°C, about 45°C to about 55°C, or about 50°C. Alternatively, step (b) may be carried out at room temperature. The stirring may be carried out for any suitable time. Typically, the stirring may be carried out over a period of about 10 minutes to about 2 hours, about 20 minutes to about 1 hour, or about 45 minutes.

In the process for preparing Form M1 as described herein, step (c) is carried out. Preferably, the cooling is to room temperature. After cooling, step (d) can be carried out, preferably by stirring the cooled mixture for a suitable time to prepare Belumosudil Mesylate Form M1. The stirring may be carried out for any suitable time, preferably for about 6 to about 96 hours, about 20 to about 80 hours, about 30 to about 50 hours, or about 40 hours.

In the process as described herein, step (e) may be carried out by any suitable method, for example by filtration, decantation or by centrifuge. Preferably the isolation of the solid is by filtration or by centrifuge, and more preferably by centrifuge.

The process as described herein may further comprise washing and/or drying steps.

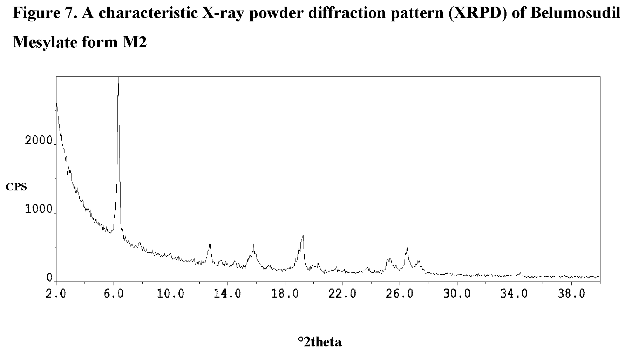

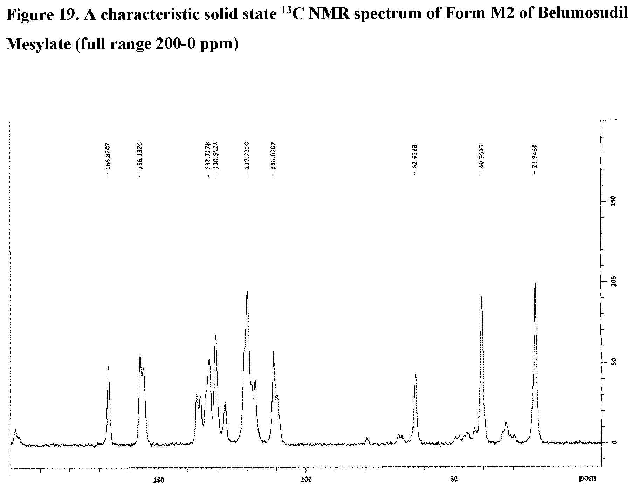

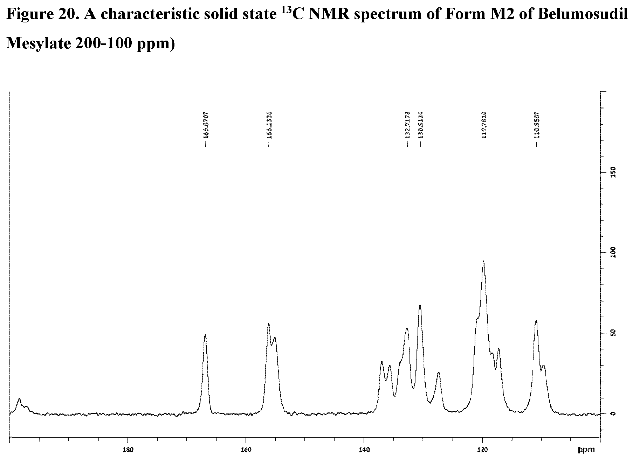

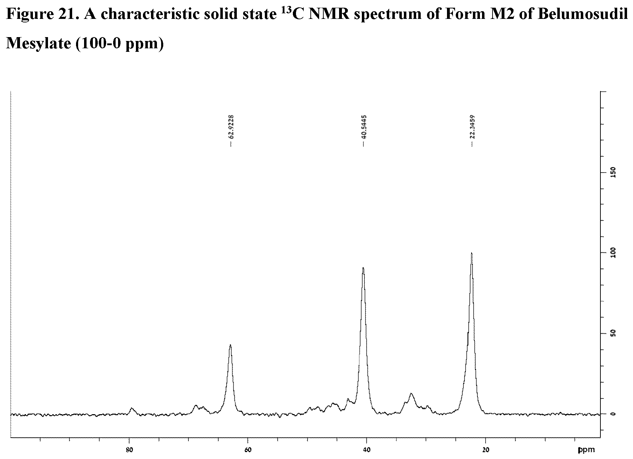

Disclosed is a crystalline polymorph of Belumosudil Mesylate, designated form M2. The crystalline Form M2 of Belumosudil Mesylate may be characterized by data selected from one or more of the following: an X-ray powder diffraction pattern substantially as depicted in Figure 7; an X-ray powder diffraction pattern having peaks at 6.3, 12.8, 15.8, 19.3 and 26.5 degrees 2-theta ± 0.2 degrees 2-theta; a solid state ¹³C NMR spectrum with characteristic peaks at 156.1, 132.7, 135.5, 119.8 and 110.9 ppm ± 0.2 ppm; a solid state ¹³C NMR spectrum having the following chemical shift absolute differences from reference peak at 166.9 ppm ± 1 ppm: 10.8, 34.2, 36.4, 47.1 and 56.0 ppm ± 0.1 ppm; a solid state ¹³C NMR spectrum substantially as depicted in any of Figures 19, 20 and 21; and combinations of these data.

Crystalline Form M2 of Belumosudil Mesylate may be further characterized by an X-ray powder diffraction pattern having peaks at 6.3, 12.8, 15.8, 19.3 and 26.5 degrees 2-theta ± 0.2 degrees 2-theta, and also having any one, two, three, four or five additional peaks selected from 7.8, 20.4, 23.7, 25.1 and 27.4 degrees 2-theta ± 0.2 degrees 2-theta.

Crystalline form M2 of Belumosudil Mesylate may be alternatively characterized by X-ray powder diffraction pattern having peaks at 6.3, 7.8, 12.8, 15.8, 19.3, 20.4, 23.7, 25.1, 26.5 and 27.4 degrees 2-theta ± 0.2 degrees 2-theta.

Crystalline form M2 of Belumosudil Mesylate may be a hydrate, preferably a dihydrate. Alternatively, crystalline form M2 of Belumosudil Mesylate may contain about 1 % to about 7 % of water, preferably about 1.5 to about 6.1 % of water by weight.

Crystalline form M2 of Belumosudil Mesylate may be isolated.

Crystalline Form M2 of Belumosudil Mesylate may be non-hygroscopic. Particularly, Form M2 of Belumosudil Mesylate may be polymorphically stable at up to 100% relative humidity at room temperature for at least 7 days.

Crystalline Form M2 of Belumosudil Mesylate may be characterized by each of the above characteristics alone/or by all possible combinations, e.g., an XRPD pattern having peaks at 6.3, 12.8, 15.8, 19.3 and 26.5 degrees 2-theta ± 0.2 degrees 2-theta; an XRPD pattern as depicted in Figure 7, and combinations thereof.

Crystalline form M2 of Belumosudil Mesylate may be prepared by crystallisation from a mixture comprising Belumosudil Mesylate and water, and optionally one or more polar organic solvents, preferably alcohols or mixtures thereof. Examples of suitable solvents include but are not limited to methanol, isopropanol, 1-propanol, n-butanol . The water may be present in an amount of: about 1 to about 60 ml per mmol of Belumosudil. The process may comprise:
(a) providing a mixture of Belumosudil Mesylate in water and optionally one or more polar organic solvents, optionally with heating,
(b) optionally stirring the mixture, optionally at elevated temperature;
(c) optionally cooling the mixture;
(d) optionally stirring the cooled mixture; and
(e) optionally isolating crystalline form M2 of Belumosudil Mesylate from the mixture.

In the process as described herein, the mixture in step (a) may be prepared by:
(i) providing a mixture of Belumosudil free base in water or a mixture of water and at least one polar organic solvent;
(ii) combining the mixture with Methanesulfonic acid; and
(iii) optionally heating the mixture.

In the process for preparing Form M2 of Belumosudil Mesylate as described herein, the polar organic solvent in step (i) may preferably comprise an alcohol or mixtures thereof. Preferably the polar organic solvent in step (i) is selected from: methanol, isopropanol, 1-propanol and n-butanol, or mixtures thereof; more preferably the polar organic solvent in step (i) is methanol, ethanol, isopropanol, 1-propanol, or n-butanol, and most preferably ethanol.

In the process for preparing Form M2 of Belumosudil Mesylate as described herein, the ratio of solvent to Belumosudil in step (i) may be: about 10 to about 40 ml per gram of Belumosudil, about 14 to about 35 ml per gram of Belumosudil or about 16 to about 28 ml per gram of Belumosudil, or about 18 to about 24 ml per gram of Belumosudil, and optionally about 20 ml per gram of Belumosudil. The mixture in step (i) may be a solution or a slurry.

In the process for preparing Form M2 of Belumosudil Mesylate as described herein, in step (ii), the combining of Methanesulfonic acid to Belumosudil may be in any order. Preferably, methanesulfonic acid may be added to the mixture of Belumosudil in the solvent. The addition may be carried out portion-wise or dropwise. The Methanesulfonic acid is optionally added in an amount of: about 0.7 to about 1.5 molar equivalents, about 0.9 to about 1.3 molar equivalents, about 1.0 to about 1.2 molar equivalents, or about 1.1 molar equivalents relative to Belumosudil. Preferably, step (ii) comprises adding Methanesulfonic acid, to the mixture of Belumosudil and solvent.

In step (iii) of the process for preparing Belumosudil Form M2, the mixture may be optionally heated. The heating may be to a temperature of about 30°C to about 70°C, about 40°C to about 60°C, about 45°C to about 55°C, or about 50°C. The heating may take place over any suitable period of time to form a solution of Belumosudil Mesylate.

Alternatively, in the process for preparing Belumosudil Form M2 as described herein, the mixture in step (a) may be prepared by a process comprising:
(i-b) combining Belumosudil Mesylate with a polar organic solvent, optionally with heating to produce a solution; and
(ii-b) adding water as antisolvent.

In the process for preparing Form M2 of Belumosudil Mesylate as described herein, the polar organic solvent in step (i-b) is preferably selected from the group consisting of TFE, DMSO and or mixtures thereof. Particularly, the solvent is selected from the group consisting of TFE and DMSO. The ratio of polar organic solvent in step (i-b) is preferably about 5 ml to 60 ml, about 7 ml to about 50 ml, about 10 ml to about 45 ml, or about 10 ml to about 40 ml, per gram of Belumosudil Mesylate.

In the process for preparing Belumosudil Form M2 as described herein, the mixture in step (a) may be prepared by a process comprising: (i-c) combining Belumosudil Mesylate with water to produce a slurry. Preferably, the Belumosudil Mesylate is Form M1 as described herein. Preferably, the ratio of water step (i-c) is preferably about 10 ml to 60 ml, about 20 ml to about 50 ml, about 30 ml to about 45 ml, or about 40 ml, per gram of Belumosudil Mesylate.

In the process as described herein, step (b) may be carried out. Step (b) is preferably carried out under heating, preferably at a temperature of: about 30°C to about 80°C, about 40°C to about 75°C, about 45°C to about 70°C, about 45°C to about 65°C, about 45°C to about 65 °C, about 48 °C to about 62°C, or about 50°C to about 60°C. The stirring may be carried out for any suitable period of time.

In the process for preparing Form M2 as described herein, step (c) is carried out. Step (c) preferably comprises cooling the mixture to room temperature.

In the process for preparing Form M2 as described herein, step (d) may be carried out. The stirring may be for any suitable time to prepare Form M2.

In the process for preparing crystalline Belumosudil Mesylate Form M2 as described herein, step (e) may be carried out by any suitable method, for example by filtration, decantation or by centrifuge. Preferably the isolation of the solid is by filtration or by centrifuge, and more preferably by centrifuge.

The process may further comprise washing and/or drying steps.

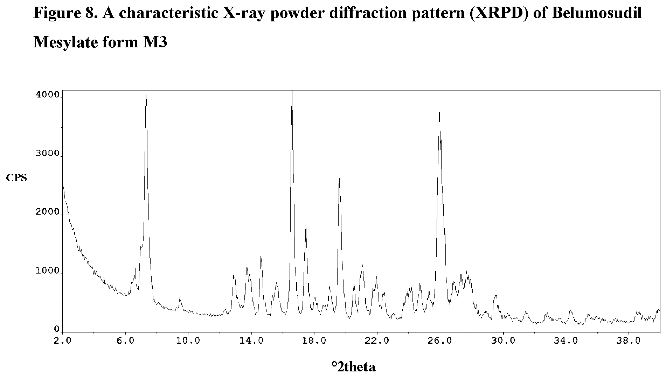

The present disclosure includes a crystalline polymorph of Belumosudil Mesylate, designated form M3. The crystalline Form M3 of Belumosudil Mesylate may be characterized by data selected from one or more of the following: an X-ray powder diffraction pattern substantially as depicted in Figure 8; an X-ray powder diffraction pattern having peaks at 7.3, 14.6, 16.6, 17.5 and 19.6 degrees 2-theta ± 0.2 degrees 2-theta; and combinations of these data.

Crystalline Form M3 of Belumosudil Mesylate may be further characterized by an X-ray powder diffraction pattern having peaks at 7.3, 14.6, 16.6, 17.5 and 19.6 degrees 2-theta ± 0.2 degrees 2-theta, and also having any one, two, three, four or five additional peaks selected from 12.9, 13.7, 19.0, 20.6 and 26.0 degrees 2-theta ± 0.2 degrees 2-theta.

Crystalline form M3 of Belumosudil Mesylate may be alternatively characterized by X-ray powder diffraction pattern having peaks at 7.3, 12.9, 13.7, 14.6, 16.6, 17.5, 19.0, 19.6, 20.6 and 26.0 degrees 2-theta ± 0.2 degrees 2-theta.

In one embodiment of the present disclosure, crystalline Form M3 of Belumosudil Mesylate is isolated.

Crystalline Form M3 of Belumosudil Mesylate may be characterized by each of the above characteristics alone/or by all possible combinations, e.g., an XRPD pattern having peaks at 7.3, 14.6, 16.6, 17.5 and 19.6 degrees 2-theta ± 0.2 degrees 2-theta; an XRPD pattern as depicted in Figure 8, and combinations thereof.

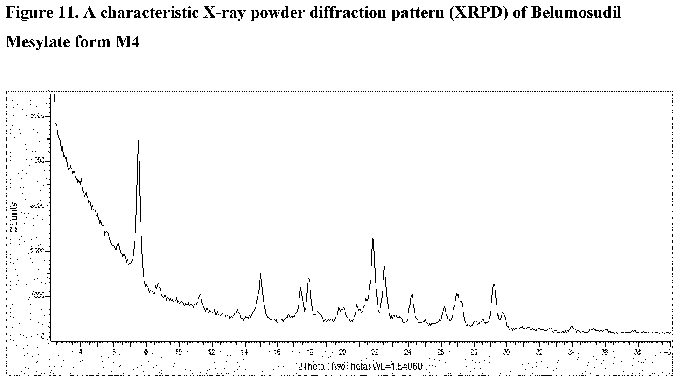

Disclosed is a crystalline polymorph of Belumosudil Mesylate, designated form M4. The crystalline Form M4 of Belumosudil Mesylate may be characterized by data selected from one or more of the following: an X-ray powder diffraction pattern substantially as depicted in Figure 11; an X-ray powder diffraction pattern having peaks at 7.5, 15.0, 17.9, 21.8 and 22.6 degrees 2-theta ± 0.2 degrees 2-theta; and combinations of these data.

Crystalline Form M4 of Belumosudil Mesylate may be further characterized by an X-ray powder diffraction pattern having peaks at 7.5, 15.0, 17.9, 21.8 and 22.6 degrees 2-theta ± 0.2 degrees 2-theta, and also having any one, two, three, four or five additional peaks selected from 11.3, 17.4, 20.9, 24.2 and 29.2 degrees 2-theta ± 0.2 degrees 2-theta.

Crystalline form M4 of Belumosudil Mesylate may be alternatively characterized by X-ray powder diffraction pattern having peaks at 7.5, 11.3, 15.0, 17.4, 17.9, 20.9, 21.8, 22.6, 24.2 and 29.2 degrees 2-theta ± 0.2 degrees 2-theta.

Crystalline Form M4 of Belumosudil Mesylate may be isolated.

Crystalline Form M4 of Belumosudil Mesylate may be characterized by each of the above characteristics alone/or by all possible combinations, e.g., an XRPD pattern having peaks at 7.5, 15.0, 17.9, 21.8 and 22.6 degrees 2-theta ± 0.2 degrees 2-theta; an XRPD pattern as depicted in Figure 11, and combinations thereof.

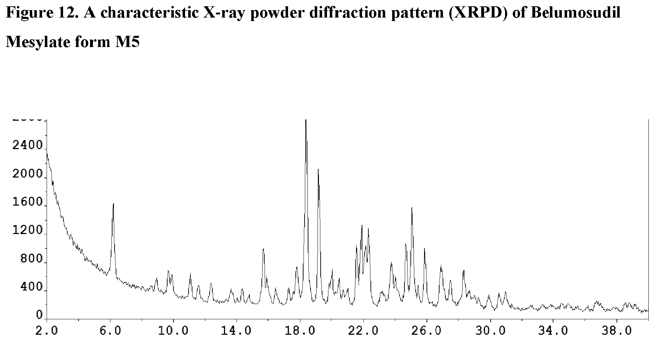

Disclosed is a crystalline polymorph of Belumosudil Mesylate, designated form M5. The crystalline Form M5 of Belumosudil Mesylate may be characterized by data selected from one or more of the following: an X-ray powder diffraction pattern substantially as depicted in Figure 12; an X-ray powder diffraction pattern having peaks at 6.2, 15.7, 18.4, 19.2 and 25.1 degrees 2-theta ± 0.2 degrees 2-theta; and combinations of these data.

Crystalline Form M5 of Belumosudil Mesylate may be further characterized by an X-ray powder diffraction pattern having peaks at 6.2, 15.7, 18.4, 19.2 and 25.1 degrees 2-theta ± 0.2 degrees 2-theta, and also having any one, two, three, four or five additional peaks selected from 17.8, 21.6, 21.9, 24.7, 25.9 degrees 2-theta ± 0.2 degrees 2-theta.

Crystalline form M5 of Belumosudil Mesylate may be alternatively characterized by X-ray powder diffraction pattern having peaks at 6.2, 15.7, 17.8, 18.4, 19.2, 21.6, 21.9, 24.7, 25.1 and 25.9 degrees 2-theta ± 0.2 degrees 2-theta.

Crystalline Form M5 of Belumosudil Mesylate may be isolated.

Crystalline Form M5 of Belumosudil Mesylate may be characterized by each of the above characteristics alone/or by all possible combinations, e.g., an XRPD pattern having peaks at 6.2, 15.7, 18.4, 19.2 and 25.1 degrees 2-theta ± 0.2 degrees 2-theta; an XRPD pattern as depicted in Figure 12, and combinations thereof.

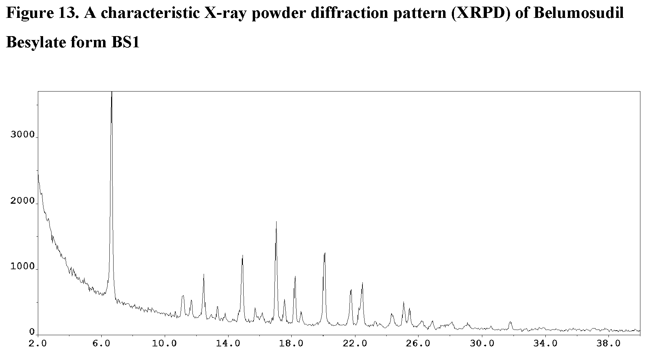

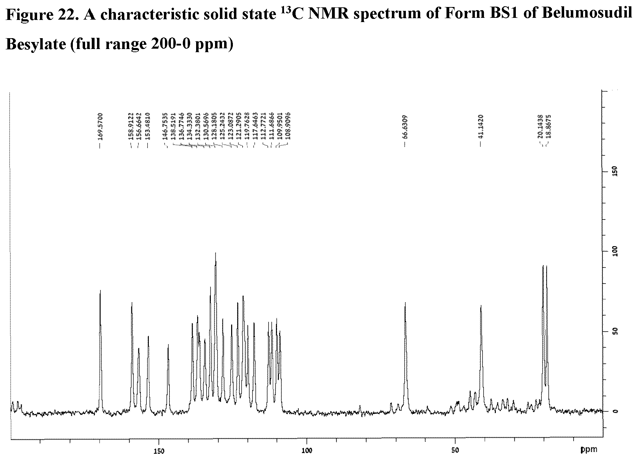

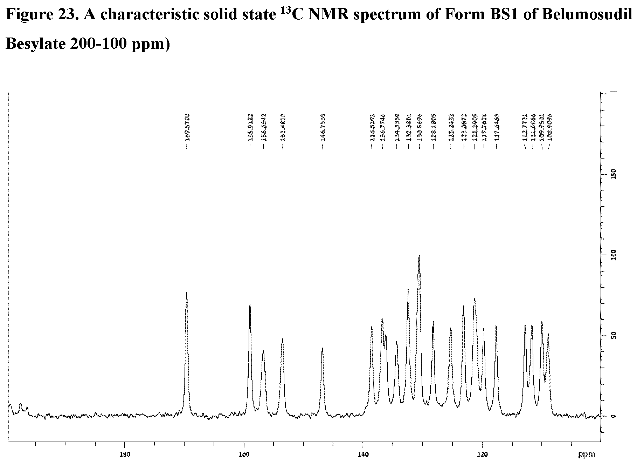

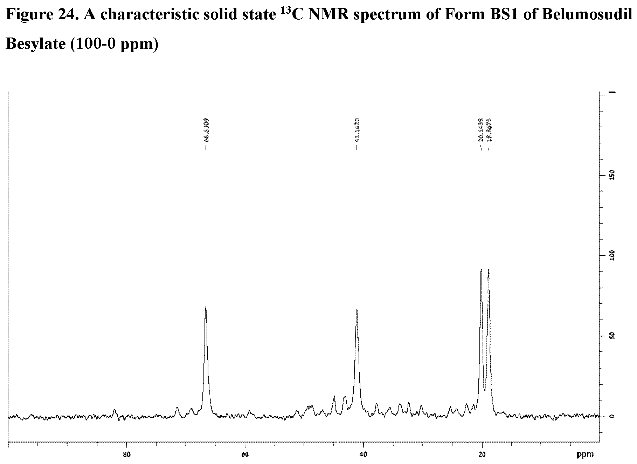

Disclosed is a crystalline polymorph of Belumosudil Besylate, designated form BS1. The crystalline Form BS1 of Belumosudil Besylate may be characterized by data selected from one or more of the following: an X-ray powder diffraction pattern substantially as depicted in Figure 13; an X-ray powder diffraction pattern having peaks at 6.6, 12.5, 14.9, 17.0 and 20.1 degrees 2-theta ± 0.2 degrees 2-theta; a solid state ¹³C NMR spectrum with characteristic peaks at 158.9, 146.8, 134.3, 121.3 and 117.6 ppm ± 0.2 ppm; a solid state ¹³C NMR spectrum having the following chemical shift absolute differences from reference peak at 169.6 ppm ± 1 ppm: 10.7, 22.8, 35.3, 48.3 and 52.0 ppm ± 0.1 ppm; a solid state ¹³C NMR spectrum substantially as depicted in any of Figures 22, 23 and 24 and combinations of these data.

Crystalline Form BS1 of Belumosudil Besylate may be further characterized by an X-ray powder diffraction pattern having peaks at 6.6, 12.5, 14.9, 17.0 and 20.1 degrees 2-theta ± 0.2 degrees 2-theta, and also having any one, two, three, four or five additional peaks selected from 11.6, 17.6, 18.2, 21.7 and 25.1 degrees 2-theta ± 0.2 degrees 2-theta.

Crystalline form BS1 of Belumosudil Besylate may be alternatively characterized by X-ray powder diffraction pattern having peaks at 6.6, 11.6, 12.5, 14.9, 17.0, 17.6, 18.2, 20.1, 21.7 and 25.1 degrees 2-theta ± 0.2 degrees 2-theta.

Crystalline form BS1 of Belumosudil Besylate may be an anhydrous form, as can be determined by TGA. The present disclosure comprises crystalline form BS1 of Belumosudil Besylate having 0.5% w/w or less, or 0.2% w/w or less, or 0.1 wt% or less, of total residual solvent. Crystalline form BS1 of Belumosudil Besylate may contain 0.5% w/w or less, or 0.2% w/w or less, or 0.1 wt% or less, of total residual solvent. The residual organic solvent may be one or more polar solvents, preferably water, alcohols (particularly C₁₋₄ alcohols, especially ethanol, methanol, isopropanol, 1-propanol, or n-butanol), and most preferably the residual organic solvent may be ethanol. Particularly, crystalline form BS1 of Belumosudil Besylate may contain 0.5% w/w or less, or 0.2 w/w or less, or 0.1 wt% or less, of ethanol.

Crystalline form BS1 of Belumosudil Besylate may be isolated.

Crystalline Form BS1 of Belumosudil Mesylate may be non-hygroscopic. Particularly, Form BS1 of Belumosudil Mesylate may be polymorphically stable at up to 100% relative humidity at room temperature for at least 7 days.

Crystalline Form BS1 of Belumosudil Besylate may be characterized by each of the above characteristics alone/or by all possible combinations, e.g., an XRPD pattern having peaks at 6.6, 12.5, 14.9, 17.0 and 20.1 degrees 2-theta ± 0.2 degrees 2-theta; an XRPD pattern as depicted in Figure 13, and combinations thereof.

Crystalline form BS1 of Belumosudil Besylate may be prepared by crystallisation from a mixture comprising Belumosudil Besylate and a polar solvent such as ethanol. In The process may comprise:
(a) providing a mixture of Belumosudil Besylate in one or more polar solvents;
(b) optionally stirring the mixture, optionally at elevated temperature;
(c) optionally cooling the mixture; and
(d) optionally isolating crystalline form BS1 of Belumosudil Besylate from the mixture.

In the process for preparing Form BS1 as described herein, the mixture in step (a) may be prepared by:
(i) providing a mixture of Belumosudil free base (preferably Form B1 as described herein) in a polar solvent; and
(ii) combining the mixture with Benzenesulfonic acid.

In the process for preparing Form BS1 as described herein, the polar solvent in step (i) is preferably an alcohol (particularly C₁₋₄ alcohols, especially ethanol, methanol, isopropanol, 1-propanol, or n-butanol). Preferably the polar solvent comprises ethanol or isopropanol and more preferably the polar solvent is ethanol. In step (i), the ratio of solvent to Belumosudil may be: about 10 to about 40 ml per gram of Belumosudil, about 14 to about 35 ml per gram of Belumosudil or about 16 to about 28 ml per gram of Belumosudil, or about 18 to about 24 ml per gram of Belumosudil, and optionally about 20 ml per gram of Belumosudil. The mixture may be a solution or a slurry.

In the process for preparing Form BS1 as described herein, step (ii) comprises combining Benzenesulfonic acid to Belumosudil. The combining may be in any order. Preferably, benzenesulfonic acid may be added to the mixture of Belumosudil in the solvent. The addition may be carried out portion-wise or dropwise. The Benzenesulfonic acid is optionally added in an amount of: about 0.7 to about 1.5 molar equivalents, about 0.9 to about 1.3 molar equivalents, about 1.0 to about 1.2 molar equivalents, or about 1.1 molar equivalents relative to Belumosudil. Preferably, step (ii) comprises adding Benzenesulfonic acid to the mixture of Belumosudil and polar solvent.

Alternatively, in the process for preparing Form BS1 as described herein, the mixture in step (a) may be prepared by combining Belumosudil Besylate with a polar solvent. Preferably the polar solvent is an alcohol (particularly C₁₋₄ alcohols, especially ethanol, methanol, isopropanol, 1-propanol, or n-butanol), and most preferably the polar solvent is ethanol.

In the process for preparing form BS1 as described herein, step (b) may be carried out, preferably by stirring at room temperature, more preferably at a temperature of: about 30°C to about 70°C, about 40°C to about 60°C, about 45°C to about 55°C, or about 50°C. Preferably, the stirring is carried out at a temperature. The stirring may be carried out for any suitable time to form Belumosudil Besylate Form BS1. Typically, the stirring may be carried out over a period of about 10 minutes to about 2 hours, about 20 minutes to about 1 hour, or about 45 minutes.

In the process for preparing form BS1 as described herein, step (c) is carried out, preferably wherein the cooling is to room temperature.

In the process for preparing form BS1 as described herein, step (d) may be carried out by any suitable method, for example by filtration, decantation or by centrifuge. Preferably the isolation of the solid is by filtration or by centrifuge, and more preferably by centrifuge.

The process for preparing from BS1 as described herein may further comprise washing and/or drying steps.

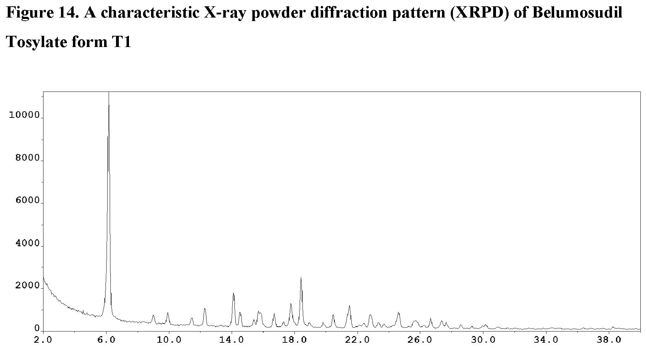

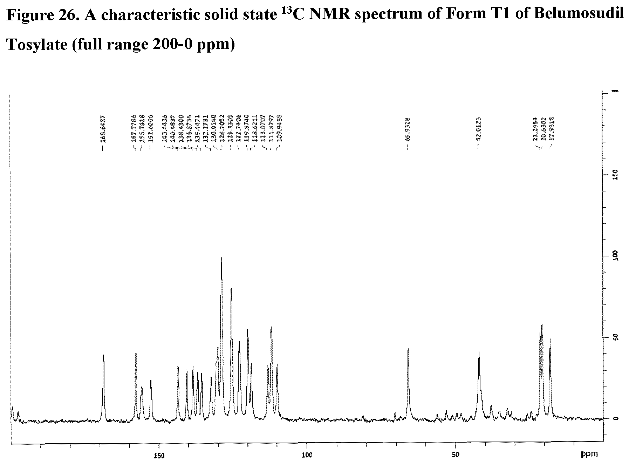

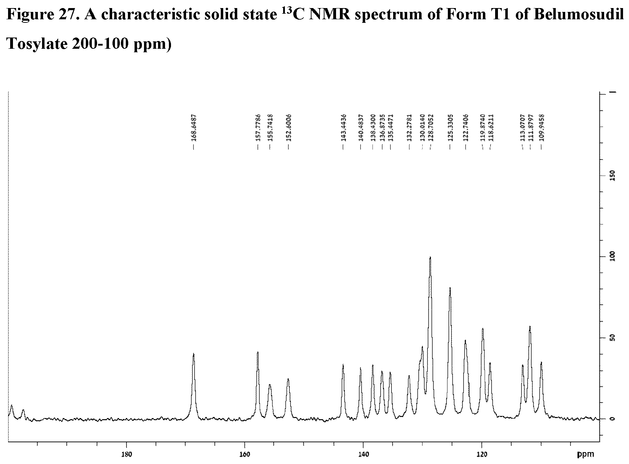

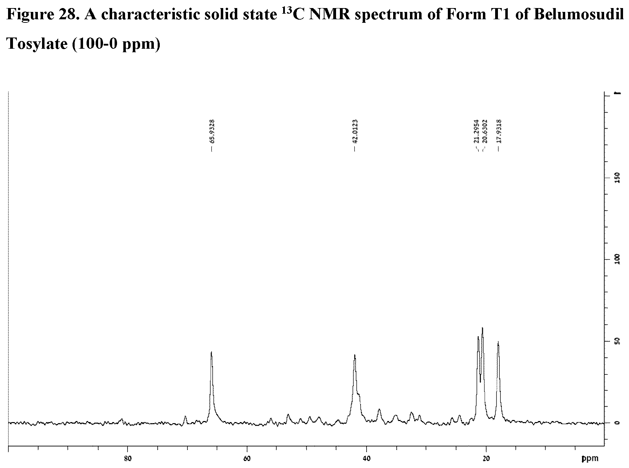

Disclosed is a crystalline polymorph of Belumosudil Tosylate, designated form T1. The crystalline Form T1 of Belumosudil Tosylate may be characterized by data selected from one or more of the following: an X-ray powder diffraction pattern substantially as depicted in Figure 14; an X-ray powder diffraction pattern having peaks at 6.2, 12.3, 14.1, 17.7 and 18.4 degrees 2-theta ± 0.2 degrees 2-theta; a solid state ¹³C NMR spectrum with characteristic peaks at 152.6, 143.4, 132.3, 125.3 and 119.8 ppm ± 0.2 ppm; a solid state ¹³C NMR spectrum having the following chemical shift absolute differences from reference peak at 168.6 ppm ± 1 ppm: 16.0, 25.2, 36.3, 43.3 and 48.8 ppm ± 0.1 ppm; a solid state ¹³C NMR spectrum substantially as depicted in any of Figures 26, 27 and 28; and combinations of these data.

Crystalline Form T1 of Belumosudil Tosylate may be further characterized by an X-ray powder diffraction pattern having peaks at 6.2, 12.3, 14.1, 17.7 and 18.4 degrees 2-theta ± 0.2 degrees 2-theta, and also having any one, two, three, four or five additional peaks selected from 9.9, 14.5, 16.7, 21.5 and 24.6 degrees 2-theta ± 0.2 degrees 2-theta.

Crystalline form T1 of Belumosudil Tosylate may be alternatively characterized by X-ray powder diffraction pattern having peaks at 6.2, 9.9, 12.3, 14.1, 14.5, 16.7, 17.7, 18.4, 21.5 and 24.6 degrees 2-theta ± 0.2 degrees 2-theta.

Crystalline form T1 of Belumosudil Tosylate may be an anhydrous form, as can be determined by TGA. The present disclosure comprises crystalline form T1 of Belumosudil Tosylate having 0.5% w/w or less, or 0.2% w/w or less, or 0.1 wt% or less, of total residual solvent. The residual solvent may be one or more polar solvents, preferably water and/or alcohols (particularly C₁₋₄ alcohols, especially ethanol, methanol, isopropanol, 1-propanol, or n-butanol), and most preferably the residual organic solvent may be methanol. Particularly, crystalline form T1 of Belumosudil Tosylate may contain to 0.5% w/w or less, or 0.2% w/w or less, or 0.1 wt% or less, of methanol.

Crystalline form T1 of Belumosudil Tosylate may be isolated.

Crystalline Form T1 of Belumosudil Mesylate may be non-hygroscopic. Particularly, Form T1 of Belumosudil Mesylate may be polymorphically stable at up to 100% relative humidity at room temperature for at least 7 days.

Crystalline Form T1 of Belumosudil Tosylate may be characterized by each of the above characteristics alone/or by all possible combinations, e.g., an XRPD pattern having peaks at 6.2, 12.3, 14.1, 17.7 and 18.4 degrees 2-theta ± 0.2 degrees 2-theta; an XRPD pattern as depicted in Figure 14, and combinations thereof.

Crystalline form T1 of Belumosudil Tosylate may be prepared by crystallisation from a mixture comprising Belumosudil Tosylate and one or more polar solvents such as alcohols. Preferably, the solvent is methanol. The process may comprise:
(a) providing a mixture of Belumosudil Tosylate in one or more polar solvents, preferably comprising methanol;
(b) optionally stirring the mixture, optionally at elevated temperature;
(c) optionally cooling the mixture; and
(d) optionally isolating crystalline form T1 of Belumosudil Tosylate from the mixture.

In the process as described herein, the mixture in step (a) may be prepared by:
(i) providing a mixture of Belumosudil free base in a polar solvent; and
(ii) combining the mixture with Toluenesulfonic acid.

Preferably, the process for preparing Form T1 of Belumosudil Tosylate as described herein is carried out in the absence of water or substantially in the absence of water, particularly 2 wt% or less, 1 wt% or less, 0.5 wt% or less, 0.2 wt% or less, or 0.1 wt% or less of water.

In the process for preparing Form T1 as described herein, the polar solvent is preferably an alcohol (particularly C₁₋₄ alcohols, especially ethanol, methanol, isopropanol, 1-propanol, or n-butanol), and most preferably the polar solvent is methanol. In step (i), the ratio of solvent to Belumosudil may be: about 10 to about 40 ml per gram of Belumosudil, about 14 to about 35 ml per gram of Belumosudil or about 16 to about 28 ml per gram of Belumosudil, or about 18 to about 24 ml per gram of Belumosudil, and optionally about 20 ml per gram of Belumosudil. The mixture may be a solution or a slurry

In step (ii), combining of Toluenesulfonic acid to Belumosudil may be in any order. Preferably, Toluenesulfonic acid may be added to the mixture of Belumosudil in the solvent. The addition may be carried out portion-wise or dropwise. The Toluenesulfonic acid is optionally added in an amount of: about 0.7 to about 1.5 molar equivalents, about 0.9 to about 1.3 molar equivalents, about 1.0 to about 1.2 molar equivalents, or about 1.1 molar equivalents relative to Belumosudil. Preferably, step (ii) comprises adding Toluenesulfonic acid, to the mixture of Belumosudil and solvent.

Alternatively, in the process for preparing Form T1 as described herein, the mixture in step (a) may be prepared by combining Belumosudil Tosylate with a polar solvent. Preferably the polar solvent is an alcohol (particularly C₁₋₄ alcohols, especially ethanol, methanol, isopropanol, 1-propanol, or n-butanol), and most preferably the polar solvent is methanol.

In the process for preparing Form T1 as described herein, step (b) is carried out, preferably by stirring at a temperature of: about 30°C to about 70°C, about 40°C to about 60°C, about 45°C to about 55°C, or about 50°C. The stirring may be carried out for any suitable time.

In the process for preparing form T1 as described herein, step (c) is carried out, preferably wherein the cooling is to room temperature.

In the process for preparing form T1 as described herein, step (d) may be carried out by any suitable method, for example by filtration, decantation or by centrifuge. Preferably the isolation of the solid is by filtration or by centrifuge, and more preferably by centrifuge.

In the process for preparing Form T1 as described herein, the process may further comprise washing and/or drying steps.

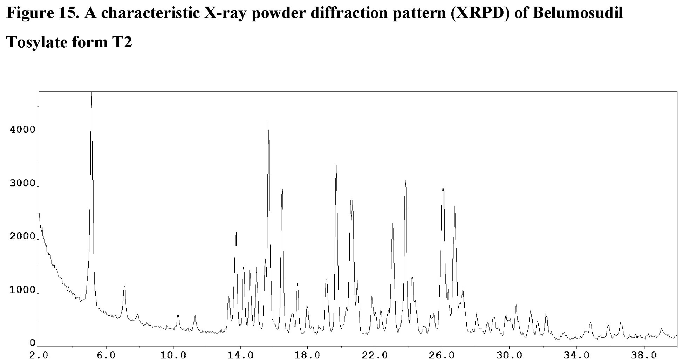

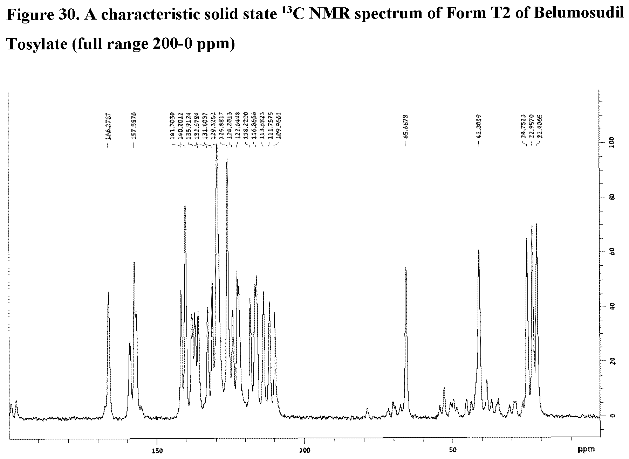

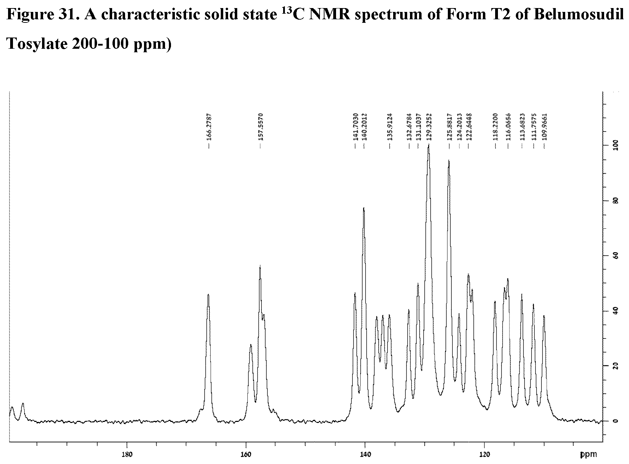

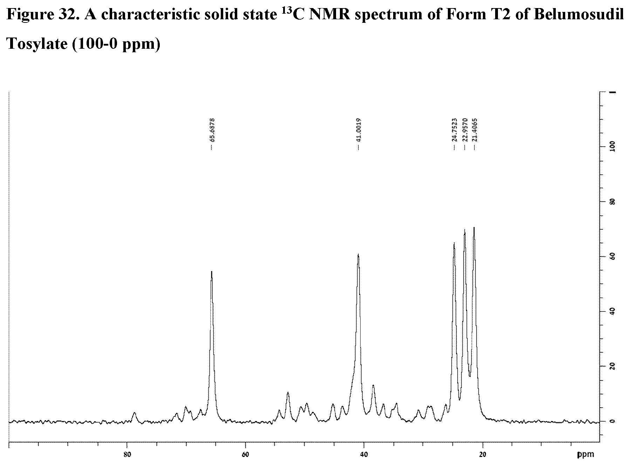

Disclosed is a crystalline polymorph of Belumosudil Tosylate, designated form T2. The crystalline Form T2 of Belumosudil Tosylate may be characterized by data selected from one or more of the following: an X-ray powder diffraction pattern substantially as depicted in Figure 15; an X-ray powder diffraction pattern having peaks at 5.1, 15.7, 16.4, 19.7 and 23.7 degrees 2-theta ± 0.2 degrees 2-theta; a solid state ¹³C NMR spectrum with characteristic peaks at 141.7, 140.2, 131.1, 125.9 and 124.2 ppm ± 0.2 ppm; a solid state ¹³C NMR spectrum having the following chemical shift absolute differences from reference peak at 166.3 ppm ± 1 ppm: 24.6, 26.1, 35.2, 40.4 and 42.1 ppm ± 0.1 ppm; a solid state ¹³C NMR spectrum substantially as depicted in any of Figures 30, 31 and 32; and combinations of these data.

Crystalline Form T2 of Belumosudil Tosylate may be further characterized by an X-ray powder diffraction pattern having peaks at 5.1, 15.7, 16.4, 19.7 and 23.7 degrees 2-theta ± 0.2 degrees 2-theta, and also having any one, two, three, four or five additional peaks selected from 13.7, 14.2, 14.6, 19.1 and 23.0 degrees 2-theta ± 0.2 degrees 2-theta.

Crystalline form T2 of Belumosudil Tosylate may be alternatively characterized by X-ray powder diffraction pattern having peaks at 5.1, 13.7, 14.2, 14.6, 15.7, 16.4, 19.1, 19.7, 23.0 and 23.7 degrees 2-theta ± 0.2 degrees 2-theta.

Crystalline form T2 of Belumosudil Tosylate may be an anhydrous form, as can be determined by TGA. The present disclosure comprises crystalline form T2 of Belumosudil Tosylate having 1% w/w or less, or 0.5% w/w or less or 0.2% w/w or less, or 0.1 wt% or less, of total residual solvent. The residual solvent may be one or more polar solvents, preferably water or alcohols (particularly C₁₋₄ alcohols, especially ethanol, methanol, isopropanol, 1-propanol, or n-butanol), and most preferably the residual organic solvent may be water and/or ethanol. Particularly, crystalline form T2 of Belumosudil Tosylate may contain 0.5% w/w or less, or 0.2% w/w or less, or 0.1 wt% or less, of ethanol. Crystalline Form T2 of Belumosudil Tosylate may be isolated.

Crystalline Form T2 of Belumosudil Mesylate may be non-hygroscopic. Particularly, Form T2 of Belumosudil Mesylate may be polymorphically stable at up to 100% relative humidity at room temperature for at least 7 days.

Crystalline Form T2 of Belumosudil Tosylate may be characterized by each of the above characteristics alone/or by all possible combinations, e.g., an XRPD pattern having peaks at 5.1, 15.7, 16.4, 19.7 and 23.7 degrees 2-theta ± 0.2 degrees 2-theta; an XRPD pattern as depicted in Figure 15, and combinations thereof.

Crystalline form T2 of Belumosudil Tosylate may be prepared by crystallisation from a mixture comprising Belumosudil Tosylate and one or more polar solvent such as water. The process may comprise:
(a) providing a mixture of Belumosudil Tosylate in one or more polar solvent;
(b) optionally stirring the mixture, optionally at elevated temperature;
(c) optionally cooling to room temperature; and
(d) optionally isolating crystalline form T2 of Belumosudil Mesylate from the mixture.

In the process for preparing Form T2 as described herein, the mixture in step (a) may be prepared by:
(i) providing a mixture of Belumosudil free base (preferably Form B1 as described herein) in a polar solvent, preferably comprising water and/or ethanol; and
(ii) combining the mixture with Toluenesulfonic acid, optionally at elevated temperature; and
(iii) optionally adding a polar solvent.

In the process for preparing Form T2 as described herein, the polar solvent in step (i) preferably comprises water, and more preferably the polar solvent in step (i) water. In step (i), the ratio of solvent to Belumosudil may be: about 10 to about 40 ml per gram of Belumosudil, about 14 to about 35 ml per gram of Belumosudil or about 16 to about 28 ml per gram of Belumosudil, or about 18 to about 24 ml per gram of Belumosudil, about 20 to about 30, and optionally about 20 ml per gram of Belumosudil. The mixture may be a solution or a slurry.

In the process for preparing Form T2 as described herein, step (ii) comprises combining Toluenesulfonic acid to Belumosudil in any order. Preferably, Toluenesulfonic acid may be added to the mixture of Belumosudil in the solvent. The addition may be carried out portion-wise or dropwise. The Toluenesulfonic acid is optionally added in an amount of: about 0.7 to about 1.5 molar equivalents, about 0.9 to about 1.3 molar equivalents, about 1.0 to about 1.2 molar equivalents, or about 1.1 molar equivalents relative to Belumosudil. Preferably, step (ii) comprises adding Toluenesulfonic acid, to the mixture of Belumosudil and solvent.

In the process for preparing Form T2 as described herein, in step (iii), the polar solvent is preferably selected from the group consisting of methanol, ethanol, isopropanol, 1-propanol, and n-butanol. More preferably, the polar solvent in step (iii) is ethanol. In step (iii), the ratio of total solvent to Belumosudil may be from: about 20 to about 50 ml, about 20 to about 40 ml, about 22 to about 35 ml, or about 25 ml, per gram of Belumosudil.

Alternatively, in the process for preparing Form T2 as described herein, the mixture in step (a) may be prepared by combining Belumosudil Tosylate with one or more polar solvents. Preferably the polar solvent is water and/or an alcohol (particularly C₁₋₄ alcohols, especially ethanol, methanol, isopropanol, 1-propanol, or n-butanol). Preferably, the polar solvent is water and ethanol.

In the process for preparing Form T2 as described herein, step (b) is carried out, preferably by stirring at a temperature of: about 30°C to about 70°C, about 40°C to about 60°C, about 45°C to about 55°C, or about 50°C. The stirring may be carried out for any suitable time. Typically, the stirring may be carried out over a period of about 10 minutes to about 2 hours, about 20 minutes to about 1 hour, or about 50 minutes.

In the process for preparing Form T2 as described herein, the mixture may be cooled, preferably to room temperature, and step (d) may be carried out by any suitable method, for example by filtration, decantation or by centrifuge. Preferably the isolation of the solid is by filtration or by centrifuge, and more preferably by centrifuge.

The process as described herein may further comprise washing and/or drying steps.

The above crystalline polymorph Form M3 of Belumosudil Mesylate can be used to prepare other crystalline polymorphs of Belumosudil, Belumosudil salts and their solid state forms.

The present disclosure encompasses a process for preparing other solid state forms of Belumosudil, Belumosudil salts and their solid state forms thereof. The process includes preparing Belumosudil Mesylate Form M3 by the processes of the present disclosure, and converting that salt to said other Belumosudil salt. The conversion can be done, for example, by a process including basifying any one or a combination of the above described Belumosudil Mesylate Form M3, and reacting the obtained Belumosudil base with an appropriate acid, to obtain the corresponding salt. Alternatively, the conversion can be done by salt switching, i.e., reacting a Belumosudil acid addition salt, with an acid having a pKa which is lower than the pKa of the acid of the Belumosudil mesylate.

The present disclosure provides the above described crystalline polymorph Form M3 of Belumosudil Mesylate for use in the preparation of pharmaceutical compositions comprising Belumosudil, salts thereof and/or crystalline polymorphs thereof.

The present disclosure also encompasses the use of crystalline polymorph Form M3 of Belumosudil Mesylate of the present disclosure for the preparation of pharmaceutical compositions of Belumosudil Mesylate, Belumosudil Tosylate and/or Belumosudil Besylate and/or crystalline polymorphs thereof.

In any aspect or embodiment of the present disclosure, Form M3 of Belumosudil Mesylate described herein may be polymorphically pure or may be substantially free of any other solid state forms of Belumosudil Mesylate. In any aspect or embodiment of the present disclosure, Form M3 of Belumosudil Mesylate may contain: about 20% (w/w) or less, about 10% (w/w) or less, about 5% (w/w) or less, about 2% (w/w) or less, about 1% (w/w) or less, about 0.5% (w/w) or less, about 0.2% (w/w) or less, about 0.1% (w/w) or less, or about 0%, of any other solid state forms of Belumosudil Mesylate, preferably as measured by XRPD. Thus, the disclosed crystalline form M3 of Belumosudil Mesylate may be substantially free of any other solid state forms of the subject compound, and may contain greater than about 80% (w/w), greater than about 90% (w/w), greater than about 95% (w/w), greater than about 98% (w/w), greater than about 99% (w/w), or about 100% of the solid state form M3 of il, Belumosudil Mesylate.

The present disclosure includes processes for preparing the above mentioned pharmaceutical compositions. The processes include combining the crystalline polymorph Form M3 of Belumosudil Mesylate, of the present disclosure with at least one pharmaceutically acceptable excipient.

Pharmaceutical combinations or formulations of the present disclosure contain Form M3 of Belumosudil Mesylate. In addition to the active ingredient, the pharmaceutical formulations of the present disclosure can contain one or more excipients. Excipients are added to the formulation for a variety of purposes. Preferably, the pharmaceutical composition or formulation according to any aspect or embodiment of the present disclosure is in the form of a tablet or a capsule, more preferably a tablet.

Diluents increase the bulk of a solid pharmaceutical composition, and can make a pharmaceutical dosage form containing the composition easier for the patient and caregiver to handle. Diluents for solid compositions include, for example, microcrystalline cellulose (e.g., Avicel^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g., Eudragit^{®}), potassium chloride, powdered cellulose, sodium chloride, sorbitol, and talc.

Solid pharmaceutical compositions that are compacted into a dosage form, such as a tablet, can include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate, and starch.

The dissolution rate of a compacted solid pharmaceutical composition in the patient's stomach can be increased by the addition of a disintegrant to the composition. Disintegrants include alginic acid, carboxymethylcellulose calcium, carboxymethylcellulose sodium (e.g., Ac-Di-Sol^{®}, Primellose^{®}), colloidal silicon dioxide, croscarmellose sodium, crospovidone (e.g., Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g., Explotab^{®}), and starch.

Glidants can be added to improve the flowability of a non-compacted solid composition and to improve the accuracy of dosing. Excipients that can function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavoring agents and flavor enhancers make the dosage form more palatable to the patient. Common flavoring agents and flavor enhancers for pharmaceutical products that can be included in the composition of the present disclosure include maltol, vanillin, ethyl vanillin, menthol, citric acid, fumaric acid, ethyl maltol, and tartaric acid.

Solid and liquid compositions can also be dyed using any pharmaceutically acceptable colorant to improve their appearance and/or facilitate patient identification of the product and unit dosage level.

In liquid pharmaceutical compositions of the present invention, Belumosudil and any other solid excipients can be dissolved or suspended in a liquid carrier such as water, vegetable oil, alcohol, polyethylene glycol, propylene glycol, or glycerin.

Liquid pharmaceutical compositions can contain emulsifying agents to disperse uniformly throughout the composition an active ingredient or other excipient that is not soluble in the liquid carrier. Emulsifying agents that can be useful in liquid compositions of the present invention include, for example, gelatin, egg yolk, casein, cholesterol, acacia, tragacanth, chondrus, pectin, methyl cellulose, carbomer, cetostearyl alcohol, and cetyl alcohol.

Liquid pharmaceutical compositions of the present invention can also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth, xanthan gum and combinations thereof.

Sweetening agents such as sorbitol, saccharin, sodium saccharin, sucrose, aspartame, fructose, mannitol, and invert sugar can be added to improve the taste.

Preservatives and chelating agents such as alcohol, sodium benzoate, butylated hydroxyl toluene, butylated hydroxyanisole, and ethylenediamine tetraacetic acid can be added at levels safe for ingestion to improve storage stability.

According to the present disclosure, a liquid composition can also contain a buffer such as gluconic acid, lactic acid, citric acid, or acetic acid, sodium gluconate, sodium lactate, sodium citrate, or sodium acetate. Selection of excipients and the amounts used can be readily determined by the formulation scientist based upon experience and consideration of standard procedures and reference works in the field.

The solid compositions of the present disclosure include powders, granulates, aggregates, and compacted compositions. The dosages include dosages suitable for oral, buccal, rectal, parenteral (including subcutaneous, intramuscular, and intravenous), inhalant, and ophthalmic administration. Although the most suitable administration in any given case will depend on the nature and severity of the condition being treated, in embodiments the route of administration is oral. The dosages can be conveniently presented in unit dosage form and prepared by any of the methods well-known in the pharmaceutical arts.

Dosage forms include solid dosage forms like tablets, powders, capsules, suppositories, sachets, troches, and lozenges, as well as liquid syrups, suspensions, and elixirs.

The dosage form of the present disclosure can be a capsule containing the composition, such as a powdered or granulated solid composition of the disclosure, within either a hard or soft shell. The shell can be made from gelatin and optionally contain a plasticizer such as glycerin and/or sorbitol, an opacifying agent and/or colorant.

The active ingredient and excipients can be formulated into compositions and dosage forms according to methods known in the art.

A composition for tableting or capsule filling can be prepared by wet granulation. In wet granulation, some or all of the active ingredients and excipients in powder form are blended and then further mixed in the presence of a liquid, typically water, that causes the powders to clump into granules. The granulate is screened and/or milled, dried, and then screened and/or milled to the desired particle size. The granulate can then be tableted, or other excipients can be added prior to tableting, such as a glidant and/or a lubricant.

A tableting composition can be prepared conventionally by dry blending. For example, the blended composition of the actives and excipients can be compacted into a slug or a sheet and then comminuted into compacted granules. The compacted granules can subsequently be compressed into a tablet.

As an alternative to dry granulation, a blended composition can be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate, and colloidal silica. The proper use of these and other excipients in direct compression tableting is known to those in the art with experience and skill in particular formulation challenges of direct compression tableting.

A capsule filling of the present disclosure can include any of the aforementioned blends and granulates that were described with reference to tableting, but they are not subjected to a final tableting step.

A pharmaceutical formulation of Belumosudil Mesylate can be administered. Belumosudil Mesylate may be formulated for administration to a mammal, in embodiments to a human, by injection. Belumosudil can be formulated, for example, as a viscous liquid solution or suspension, such as a clear solution, for injection. The formulation can contain one or more solvents. A suitable solvent can be selected by considering the solvent's physical and chemical stability at various pH levels, viscosity (which would allow for syringeability), fluidity, boiling point, miscibility, and purity. Suitable solvents include alcohol USP, benzyl alcohol NF, benzyl benzoate USP, and Castor oil USP. Additional substances can be added to the formulation such as buffers, solubilizers, and antioxidants, among others. Ansel et al., Pharmaceutical Dosage Forms and Drug Delivery Systems, 7th ed.

The crystalline polymorph Form M3 of Belumosudil Mesylate and the pharmaceutical compositions and/or formulations of the present disclosure can be used as medicaments, in embodiments in the treatment of Graft-Versus-Host Disease including Chronic Graft-Versus-Host Disease, Systemic Sclerosis including Diffuse Cutaneous Systemic Sclerosis, Fibrosis including Idiopathic Pulmonary Fibrosis, Plaque Psoriasis, Systemic Scleroderma, and particularly Chronic Graft-Versus-Host Disease and/or Systemic Sclerosis.

The present disclosure also provides methods of treating Graft-Versus-Host Disease including Chronic Graft-Versus-Host Disease, Systemic Sclerosis including Diffuse Cutaneous Systemic Sclerosis, Fibrosis including Idiopathic Pulmonary Fibrosis, Plaque Psoriasis, Systemic Scleroderma, and particularly Chronic Graft-Versus-Host Disease and/or Systemic Sclerosis by administering a therapeutically effective amount of the crystalline polymorph Form M3 of Belumosudil Mesylate of the present disclosure, or at least one of the above pharmaceutical compositions and/or formulations, to a subject in need of the treatment.

Having thus described the disclosure with reference to particular preferred embodiments and illustrative examples, those in the art can appreciate modifications to the disclosure as described and illustrated that do not depart from the scope of the disclosure as disclosed in the claims. The Examples are set forth to aid in understanding the disclosure but are not intended to, and should not be construed to limit its scope in any way.

### Powder X-ray Diffraction ("XRPD") method

For Examples 1-9 and 12-15: XRPD analysis was performed on ARL (SCINTAG) powder X-Ray diffractometer model X'TRA equipped with a solid state detector. Copper radiation of 1.5418 Å was used. Scanning parameters: range: 2-40 degrees two-theta; scan mode: continuous scan; step size: 0.05°, and a rate of 3 deg/min.

For examples 10 and 11:_XRPD analysis was performed on a Bruker powder X-Ray diffractometer model D8 ADVANCE equipped with a solid state detector. Copper radiation of 1.54060 Å was used. Scanning parameters: range: 2-40 degrees two-theta; scan mode; step size: 0.05°.

The positions of the peaks were corrected respective to the silicon theoretical peak at 28.45 degrees two theta.

### Solid state ¹³C-NMR method

Solid-state 13C NMR spectra were recorded with variable amplitude cross polarization, magic angle spinning and high power proton decoupling using a BRUKER Avance II+ spectrometer operating at 125MHz and controlled temperature at 0°C. A probe using 4 mm o.d. zirconia rotors was employed. The operation conditions were: contact time: 2 ms; recycle delay: 5s; 1024 scans and spin rate of 11 kHz. Chemical shifts were referenced via a replacement sample of glycine (carboxyl carbon chemical shift assigned as 176.03 ppm relative to the signal of tetramethylsilane).

### TGA Method

Thermogravimetric analysis was carried out Mettler Toledo TGA/DSC with the following scanning parameters:
Heating between 25 -250 °C.
Heating rate: 10 °C/min.
Purging with 40 ml/min N₂ flow.
Sample weight: 7-15 mg.
Crucible: 150 µL alumina Crucible with standard aluminum lid.

### SEM Method

SEM micrographs were taken on Phenom Pro, scanning microscope at 10 kV, low current. Samples were sputtered with gold by Denton Desk V sputter coater.

### EXAMPLES

### Preparation of starting materials

Belumosudil can be prepared according to methods known from the literature, for example according to International Publication No. WO 2006/105081.
Examples marked with an asterisk (*) are provided for reference only.

### *Example 1: Preparation of amorphous Belumosudil

### Procedure A

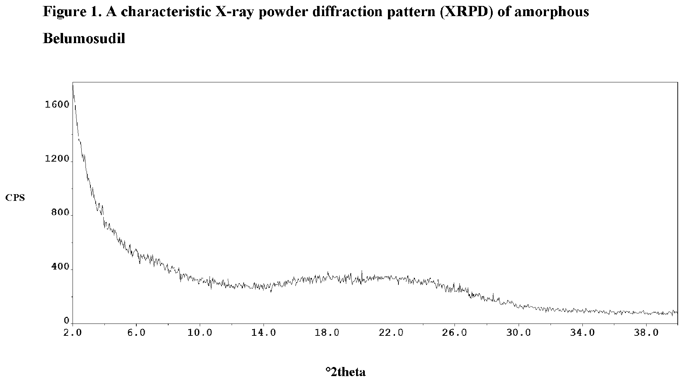

Methanol (90ml, 90V) was added to Belumosudil (1 gram, 2.21 mmol) to give a slurry. The slurry was magnetically stirred at 62°C for 15 minutes to obtain clear solution followed by mechanical filtration. The solution was cooled to room temperature and then was dried in spray drier at Tin=140°C (Tout=78°C). The obtained solid was analyzed by X-ray powder diffraction and the XRPD pattern is presented in Figure 1.

### Procedure B

Methanol (3.5 ml, 70V) was added to Belumosudil (50 mg, 0.11 mmol) to give slurry. The slurry was magnetically stirred at 60°C for 15 minutes to obtain clear solution followed by mechanical filtration. The obtained clear mother-liquor was evaporated upon 50°C/300-35 mbar to give a solid. The obtained solid was characterized by X-ray powder diffraction as Amorphous Belumosudil.

### *Example 2: Preparation of Crystalline form B1 of Belumosudil

### Procedure A

Acetone (7.5 ml, 250V) was added to Belumosudil (30 mg, 0.066 mmol) to obtain a clear solution. The solution was then mechanically filtered at room temperature and allowed to slowly evaporate at this temperature over a period of 6 days. The obtained solid was analyzed by X-ray powder diffraction and Belumosudil crystal form B1 was obtained. The XRPD pattern is presented in Figure 2.

### Procedure B

Ethanol (0.6 ml, 20V) was added to Amorphous Belumosudil (30 mg, 0.066 mmol) to give slurry. The slurry was magnetically stirred at 50 °C over a period of 5 hours. The solid was separated by centrifuge and dried in a vacuum oven at 45 °C over a period of 20 hours to afford an off white solid, which was identified as Belumosudil crystalline form B1.

### *Example 3: Preparation of Crystalline form B2 of Belumosudil

### Procedure A

Acetonitrile (12 ml, 400V) was added to Amorphous Belumosudil (30 mg, 0.066 mmol) at 80 °C to obtain clear solution. The solution was then mechanically filtered at room temperature and allowed to slowly evaporate at this temperature over a period of 5 days. The obtained solid was analyzed by XRPD and characterized as Belumosudil crystalline form B2. The XRPD pattern is presented in Figure 3.

### Procedure B

Amorphous Belumosudil (30 mg, 0.066 mmol) was heated by TGA to 160 °C over a period of 0.5 hours. The obtained solid was analyzed by X-ray powder diffraction and identified as Belumosudil crystalline form B2.

### Procedure C

Methanol (12 ml, 400V) was added to Amorphous Belumosudil (30 mg, 0.066 mmol) to give a slurry at room temperature. The slurry was magnetically stirred at 50 °C over a period of 5 hours. The solid was separated by centrifuge and dried in a vacuum oven at 45 °C over a period of 20 hours. The obtained off white solid was analyzed by X-ray powder diffraction and characterized by X-ray powder diffraction as Belumosudil crystal form B2.

### *Example 4: Preparation of Crystalline form B3 of Belumosudil

Tetrahydrofuran (5 ml, 50V) was added to Belumosudil (100 mg, 0.22 mmol) at 40 °C and stirred for 10 minutes to obtain clear solution. The solution was then mechanically filtered, cooled to room temperature and stirred over a period of 14 hours to afford a wet solid. The solid was separated by centrifuge. The obtained wet solid was analyzed by XRPD and characterized as Belumosudil crystalline form B3. The XRPD pattern is presented in Figure 4.

### *Example 5: Preparation of Crystalline form M1 of Belumosudil Mesylate

### Procedure A

Ethanol (4 ml, 20V) was added to Belumosudil (200 mg, 0. 44 mmol) to obtain a slurry at room temperature. Next, Methanesulfonic acid (31 µL, 1.1 eq) was added drop-wise to give clear solution. The solution was heated to 50 °C and precipitation was observed. Ethanol (3 ml, 15V) was added to the obtained massive precipitate followed by magnetically stirring over a period of 30 minutes at 50 °C. Next, the precipitate was spontaneously cooled to room temperature. The solid was separated by centrifuge. The obtained solid was washed twice with ethanol (400 µL, 2V) and dried in a vacuum oven at 45 °C over a period of 16 hours to afford yellow solid. The obtained solid was analyzed by X-ray powder diffraction and characterized as Belumosudil Mesylate crystal form M1. The XRPD pattern is presented in Figure 5.

### Procedure B

DMSO (0.9 ml, 30V) was added to Belumosudil Mesylate salt (30 mg, 0.06 mmol) to give slurry. The slurry was heated to 50 °C for 10 minutes to obtain complete dissolution, followed by a hot mechanical filtration. Next, ethanol as anti-solvent (2.7 ml, 100V) was added drop-wise to obtain a solid precipitate. Next, the slurry was magnetically stirred at room temperature during 40 hours. Then, the solid was isolated by centrifuge. The obtained wet solid was analyzed by X-ray powder diffraction and identified as Belumosudil Mesylate crystal form M1.

### Procedure C

Isopropyl alcohol (2 ml, 20V) was added to Belumosudil (100 mg, 0. 2 mmol) to obtain a slurry at room temperature. Next, Methanesulfonic acid (161 µL, 1.1 eq) was added drop-wise to give clear solution. The solution was heated to 50 °C and precipitation was observed and magnetically stirring over a period of 30 minutes at 50 °C. Next, the precipitate was spontaneously cooled to room temperature. The solid was separated by centrifuge. The obtained solid was washed twice with IPA (200 µL, 2V) to afford yellow solid. The obtained solid was analyzed by X-ray powder diffraction and identified as Belumosudil Mesylate crystal form M1.

### Procedure D

N-butanol (2 ml, 20V) was added to Belumosudil (100 mg, 0. 2 mmol) to obtain a slurry at room temperature. Next, Methanesulfonic acid (161 µL, 1.1 eq) was added drop-wise to give clear solution. The solution was heated to 50 °C and precipitation was observed and magnetically stirring over a period of 30 minutes at 50 °C. Next, the precipitate was spontaneously cooled to room temperature. The solid was separated by centrifuge. The obtained solid was washed twice with N-butanol (200 µL, 2V) to afford yellow solid. The obtained solid was analyzed by X-ray powder diffraction and identified as Belumosudil Mesylate crystal form M1.

### Procedure E

1-propanol (2 ml, 20V) was added to Belumosudil (100 mg, 0. 2 mmol) to obtain a slurry at room temperature. Next, Methanesulfonic acid (161 µL, 1.1 eq) was added drop-wise to give clear solution. The solution was heated to 50 °C and precipitation was observed and magnetically stirring over a period of 30 minutes at 50 °C. Next, the precipitate was spontaneously cooled to room temperature. The solid was separated by centrifuge. The obtained solid was washed twice with 1-propanol (200 µL, 2V to afford yellow solid. The obtained solid was analyzed by X-ray powder diffraction and identified as Belumosudil Mesylate crystal form M1

### Procedure F

2,2,2-Trifluoroethanol (0.3 ml, 10V) was added to Belumosudil Mesylate salt (30 mg, 0.06 mmol) to give slurry. The slurry was heated to 60 °C for 10 minutes to obtain complete dissolution, followed by a hot mechanical filtration. Next, ethanol as anti-solvent (1.2 ml, 40V) was added drop-wise to obtain a solid precipitate. Next, the slurry was magnetically stirred at room temperature during 17hours. Then, the solid was isolated by centrifuge. The obtained wet solid was analyzed by X-ray powder diffraction and identified as Belumosudil Mesylate crystal form M1.

### *Example 6: Preparation of Crystalline form B4 of Belumosudil

### Procedure A

Methanol (0.6 ml, 20V) was added to Belumosudil form B1 (30 mg, 0.066 mmol) to obtain a slurry. The slurry was then magnetically stirred at 50 °C for 5 hours. Then, the slurry was cooled to room temperature and magnetically stirred for 16 hours. The solid was then isolated at room temperature by centrifuge. The obtained wet solid was dried in a vacuum oven at 45 °C over a period of 18 hours to afford an off white solid which was characterized by X-ray powder diffraction and the XRPD pattern is presented in Figure 6.

### Procedure B

Acetonitrile:MeOH (0.75 ml, 25V, 1:1 eq) was added to B1 (50 mg, 0.11 mmol) to give a slurry. The slurry was magnetically stirred at room temperature for a period of 1 week. The solid was then isolated at room temperature by centrifuge. The obtained wet solid was characterized by X-ray powder diffraction as Belumosudil crystal form B4.

### *Example 7: Preparation of Crystalline form M2 of Belumosudil Mesylate

### Procedure A

Water (2 ml, 20V) was added to Form B1 (100 mg, 0. 22 mmol) to obtain a slurry at room temperature. Next, Methanesulfonic acid (16µL, 1.1 eq) was added drop-wise to give a slurry. The slurry was heated to 50 °C over a period of 45 minutes to obtain a clear solution. Next, the solution was spontaneously cooled to room temperature and a precipitate was observed. The obtained solid was isolated using a centrifuge. The isolated solid was washed twice with water (0.2 ml, 2V) and dried in a vacuum oven at 45 °C over a period of 18 hours to afford a yellow solid. The obtained solid was analyzed by X-ray powder diffraction and the XRPD pattern is presented in Figure 7.

### Procedure B

A Water:Ethanol (2 ml, 20V, 1:3) mixture was added to Form B1 (100 mg, 0. 22 mmol) to obtain a slurry at room temperature. Next, Methanesulfonic acid (16 µL, 1.1 eq) was added drop-wise to give a slurry. The slurry was heated to 50 °C over a period of 45 minutes to obtain a clear solution. Next, the solution was spontaneously cooled to room temperature and a precipitate was observed. The obtained solid was isolated using a centrifuge. The isolated solid was washed twice with water:ethanol (2.2 ml, 22V, 1:3) and dried in a vacuum oven at 45 °C over a period of 18 hours to afford yellow solid. The obtained solid was analyzed by X-ray powder diffraction and identified as Belumosudil Mesylate crystal form M2.

### Procedure C

Water (2ml, 40V) was added to Belumosudil Mesylate salt form M1 (50 mg, 0.11 mmol) to give a slurry. The slurry was magnetically stirred at 60°C over a period of 1 week. Then, the solid was isolated at room temperature by centrifuge. The obtained wet solid was analyzed by X-ray powder diffraction and identified as Belumosudil Mesylate crystal form M2.

### Procedure D

DMSO (2ml, 40V) was added to Belumosudil Mesylate salt form M1 (50 mg, 0.11 mmol) to give a slurry. The slurry was heated to 50 °C for 10 minutes to obtain complete dissolution, followed by a hot mechanical filtration. The solution was spontaneously cooled to room temperature and magnetically stirred for 5 days. Next, cold water as anti-solvent (5ml, 100V, 4 °C) was added drop-wise to obtain a solid precipitate. Next, the slurry was magnetically stirred at room temperature during 18 hours. Then, the solid was isolated by centrifuge. The obtained wet solid was analyzed by X-ray powder diffraction and identified as Belumosudil Mesylate crystal form M2.

### Procedure E

2,2,2-Trifluoroethanol (0.3 ml, 10V) was added to Belumosudil Mesylate salt (30 mg, 0.06 mmol) to give slurry. The slurry was heated to 60 °C for 10 minutes to obtain complete dissolution, followed by a hot mechanical filtration. Next, water as anti-solvent (1.2 ml, 40V) was added drop-wise to obtain a solid precipitate. Next, the slurry was magnetically stirred at room temperature during 17 hours. Then, the solid was isolated by centrifuge. The obtained wet solid was analyzed by X-ray powder diffraction and identified as Belumosudil Mesylate crystal form M2.

### Example 8: Preparation of Crystalline form M3 of Belumosudil Mesylate

### Procedure A

Belumosudil Mesylate salt form M2 (prepared according to procedure C of example 7) was dried in a vacuum oven at 200 °C over a period of 4 hours to afford a yellow solid. The obtained solid was characterized by X-ray powder diffraction as Belumosudil Mesylate crystal form M3.

### *Example 9: Preparation of Crystalline form B5 of Belumosudil

### Procedure A

2,2,2-Trifluoroethanol (TFE) (0.6 ml, 50V) was added to Belumosudil (12.5 mg, 0.03 mmol) to obtain clear solution. The solution was then magnetically stirred at 70 °C for 1 hour. Then, the solution was cooled to 4 °C at a rate of 20 °C every 10 minutes and a massive precipitate was observed. Additional TFE (0.1 ml, 10V) was added to give a slurry. Next, the solid was isolated using a centrifuge. Next, the mother liqueur was left for slow crystallization at 4 °C for 20 days. The obtained wet solid was filtered by Buchner and the solid was characterized by X-ray powder diffraction as Belumosudil crystal form B5 and the XRPD pattern is presented in Figure 9.

### *Example 10: Preparation of amorphous Belumosudil Mesylate

### Procedure A

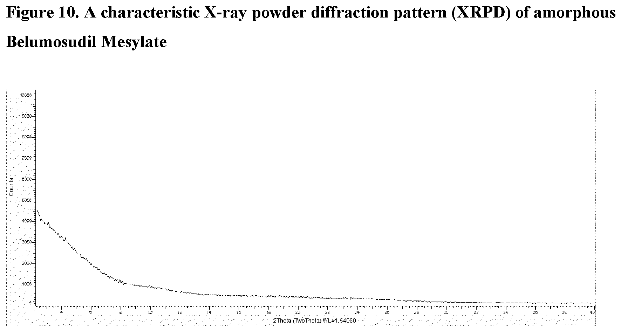

Methanol (286 ml, 220 V) was added to Belumosudil Mesylate Form M1 (1.3 grams, 2.4 mmol) to give slurry. The slurry was magnetically stirred at 62°C for 45 minutes to obtain a clear solution followed by mechanical filtration. The solution was cooled to room temperature and then was dried in spray drier at Tin=140°C (Tout=63°C). The obtained solid was analyzed by X-ray powder diffraction and identified as Amorphous Belumosudil Mesylate and the XRPD pattern is presented in Figure 10.

### *Example 11: Preparation of form M4 of Belumosudil Mesylate

### Procedure A

2, 2, 2-Trifluoroethanol (TFE) (0.7 ml, 7V) was added to Belumosudil Mesylate form M1 (100 mg, 0. 2 mmol) to obtain a clear solution. The clear solution was mechanically filtered and was added to Heptane (0.8 ml, 8V) that was precooled to 4 °C to obtain two phases. The solution was stirred at 4 °C for 4 days. Then, the mixture was seeded with Belumosudil Mesylate M3 (about 1 wt%) and after stirring for 1 day a solid precipitate was obtained. Next, the precipitate was isolated by centrifuge and dried in a vacuum oven at 45 °C over a period of 18 hours to afford yellow solid. The obtained solid was analyzed by X-ray powder diffraction and the XRPD pattern is presented in Figure 11.

### Procedure B

TFE (0.7 ml, 7V) was added to Belumosudil Mesylate form M1 (100 mg, 0. 2 mmol) to obtain a clear solution. The clear solution was mechanically filtered and was added to Heptane (0.8 ml, 8V) that was precooled to 4 °C to obtain two phases. Then, the mixture was seeded with Belumosudil Mesylate form M4 (about 1 wt%) and after stirring for 1 day solid precipitate was obtained. Next, the precipitate was isolated by centrifuge and dried in a vacuum oven at 45 °C over a period of 18 hours to afford a yellow solid. The obtained solid was analyzed by X-ray powder diffraction and identified as Belumosudil Mesylate crystal form M4.

### Procedure C

TFE (0.7 ml, 7V) was added to Belumosudil Mesylate form M1 (100 mg, 0. 2 mmol) to obtain a clear solution. The clear solution was mechanically filtered and was added to cyclopentylmethyl ether (0.8 ml, 8V) that was precooled to 4 °C. Then, the mixture was seeded with Belumosudil Mesylate form M4 (about 1 wt%) and after stirring for 18 hours a solid precipitate was obtained. Next, the precipitate was isolated by centrifuge and dried in a vacuum oven at 45 °C over a period of 18 hours to afford yellow solid. The obtained solid was analyzed by X-ray powder diffraction and identified as Belumosudil Mesylate crystal form M4.

### Procedure D

TFE (0.7 ml, 7V) was added to Belumosudil Mesylate form M1 (100 mg, 0. 2 mmol) to obtain a clear solution. The clear solution was added to cyclopentylmethyl ether (0.8 ml, 8V) that was precooled to 4 °C and massive slurry was obtained. Next, cyclopentylmethyl ether (0.4 ml, 4V) was added to obtain slurry. Then, the mixture was seeded with NaCl (about 1 wt%) and after stirring for 1 hour precipitate was obtained. Next, the precipitate was isolated by centrifuge and dried in a vacuum oven at 45 °C over a period of 18 hours to afford yellow solid. The obtained solid was analyzed by X-ray powder diffraction and identified as Belumosudil Mesylate crystal form M4.

### *Example 12: Preparation of form M5 of Belumosudil Mesylate

### Procedure A

DMSO (9 ml, 30V) was added to Belumosudil Mesylate form M1 (300 mg, 0. 6 mmol) to obtain a clear solution. Next, the clear solution was added to cyclopentylmethyl ether (9 ml, 30V) containing seeds of Belumosudil Mesylate form M4 (about 1 wt%) that was precooled to about 4 °C, then additional cyclopentylmethyl ether (18 ml, 60V) and seeds of Belumosudil Mesylate form M4 (about 1 wt%) were added and after 10 minutes some precipitant was observed. The mixture was stirred for a period of 18 hours at about 4 °C and massive precipitate was obtained. Next, the precipitate was isolated by centrifuge and dried in a vacuum oven at 45 °C over a period of 18 hours to afford yellow solid. The obtained solid was analyzed by X-ray powder diffraction and the XRPD pattern is presented in Figure 12.

### *Example 13: Preparation of form BS1 of Belumosudil Besylate

### Procedure A

Ethanol (10 ml, 20V) was added to Belumosudil form B1 (500 mg, 1.1 mmol) to obtain a slurry at room temperature. Next, Benzenesulfonic acid (192.3 mg, 1.1 eq) was added and the slurry was heated to 50 °C over a period of 45 minutes with stirring. Next, the slurry was spontaneously cooled to room temperature and the obtained solid was isolated using centrifuge. The isolated solid was dried in a vacuum oven at 45 °C over a period of 18 hours to afford yellow solid. The obtained solid was analyzed by X-ray powder diffraction and the XRPD pattern is presented in Figure 13.

### *Example 14: Preparation of form T1 of Belumosudil Tosylate

### Procedure A

Methanol (2 ml, 20V) was added to Belumosudil form B1 (100 mg, 0.22 mmol) and heated to 50 °C to obtain a slurry. Next, p-toluenesulfonic acid (46.2 mg, 1.1 eq) was added and a clear solution was obtained. After stirring for 10 minutes at 50 °C precipitation was observed. The solution was stirred at 50 °C over a period of 45 minutes. Next, the solution was spontaneously cooled to room temperature and massive precipitation was observed. The obtained solid was isolated using centrifuge and washed once with Methanol (200 µL, 2V). The solid was dried in a vacuum oven at 45 °C over a period of 18 hours to afford yellow solid. The obtained solid was analyzed by X-ray powder diffraction and the XRPD pattern is presented in Figure 14.

### *Example 15: Preparation of form T2 of Belumosudil Tosylate

### Procedure A

Water (1 ml, 20V) was added to Belumosudil form B1 (50 mg, 0.11 mmol) and heated to 50 °C to obtain slurry. Next, p-toluenesulfonic acid (23.1 mg, 1.1 eq) was added and the sticky slurry was stirred at 50 °C over a period of 20 minutes. Then, ethanol (250 µl, 5V) was added to obtain a slurry. The slurry was stirred at 50 °C over a period of 50 minutes. Next, the slurry was spontaneously cooled to room temperature and the obtained solid was isolated using centrifuge. The isolated solid was washed once with Water:Ethanol (1 ml, 2V, 4:1) and dried in a vacuum oven at 45 °C over a period of 18 hours to afford yellow solid. The obtained solid was analyzed by X-ray powder diffraction and the XRPD pattern is presented in Figure 15. 1.

## Claims

1. A crystalline form of Belumosudil Mesylate, designated form M3, which is **characterized by** an X-ray powder diffraction pattern having peaks at 7.3, 14.6, 16.6, 17.5 and 19.6 degrees 2-theta ± 0.2 degrees 2-theta.

2. A crystalline form of Belumosudil Mesylate according to Claim 1 which is **characterized by** an X-ray powder diffraction pattern having peaks at 7.3, 14.6, 16.6, 17.5 and 19.6 degrees 2-theta ± 0.2 degrees 2-theta, and also having any one, two, three, four or five additional peaks selected from 12.9, 13.7, 19.0, 20.6 and 26.0 degrees 2-theta ± 0.2 degrees 2-theta.

3. A crystalline form of Belumosudil Mesylate according to Claim 1 or Claim 2, which is **characterized by** X-ray powder diffraction pattern having peaks at 7.3, 12.9, 13.7, 14.6, 16.6, 17.5, 19.0, 19.6, 20.6 and 26.0 degrees 2-theta ± 0.2 degrees 2-theta, or an X-ray powder pattern substantially as depicted in Figure 8.

4. A crystalline form of Belumosudil Mesylate according to any of Claims 1 to 3, which is isolated.

5. A crystalline form according to any of preceding claim, which contains no more than about 20%, no more than about 10%, no more than about 5%, no more than about 2%, no more than about 1% or about 0% of any other solid state form of Belumosudil Mesylate.

6. Use of a crystalline form according to any preceding claim for the preparation of a pharmaceutical composition.

7. A pharmaceutical composition comprising a crystalline form according to any of Claims 1 to 5, and at least one pharmaceutically acceptable excipient.

8. A process for preparing a pharmaceutical composition according to Claim 7, comprising combining a crystalline form according to any of Claims 1 to 5, with at least one pharmaceutically acceptable excipient.

9. A crystalline form according to any of Claims 1 to 5, or a pharmaceutical composition according to Claim 7, for use as a medicament.

10. A crystalline form according to any of Claims 1 to 5, or a pharmaceutical composition according to Claim 7, for use in the treatment of Graft-Versus-Host Disease including Chronic Graft-Versus-Host Disease, Systemic Sclerosis including Diffuse Cutaneous Systemic Sclerosis, Fibrosis including Idiopathic Pulmonary Fibrosis, Plaque Psoriasis, Systemic Scleroderma, and particularly Chronic Graft-Versus-Host Disease and Systemic Sclerosis.

11. Use of crystalline form according to any one of Claims 1 to 5, in the preparation of another solid state form of Belumosudil, or another salt of Belumosudil or a solid state form thereof.

12. A process for preparing a Belumosudil salt or a solid state form thereof, comprising preparing crystalline form of any of Claims 1 to 5, and converting it to another solid state form of Belumosudil or another salt or a solid state form thereof.

## Patentansprüche

1. Kristalline Form von Belumosudilmesylat, die als Form M3 bezeichnet wird, die durch ein Röntgenpulverbeugungsmuster mit Peaks bei 7,3, 14,6, 16,6, 17,5 und 19,6 Grad 2-Theta ± 0,2 Grad 2-Theta gekennzeichnet ist.

2. Kristalline Form von Belumosudilmesylat nach Anspruch 1, die durch ein Röntgenpulverbeugungsmuster mit Peaks bei 7,3, 14,6, 16,6, 17,5 und 19,6 Grad 2-Theta ± 0,2 Grad 2-Theta gekennzeichnet ist und außerdem einen, zwei, drei, vier oder fünf zusätzliche Peaks, die aus 12,9, 13,7, 19,0, 20,6 und 26,0 Grad 2-Theta ± 0,2 Grad 2-Theta ausgewählt sind, aufweist.

3. Kristalline Form von Belumosudilmesylat nach Anspruch 1 oder Anspruch 2, die durch ein Röntgenpulverbeugungsmuster mit Peaks bei 7,3, 12,9, 13,7, 14,6, 16,6, 17,5, 19,0, 19,6, 20,6 und 26,0 Grad 2-Theta ± 0,2 Grad 2-Theta oder ein Röntgenpulverbeugungsmuster, das weitgehend der Darstellung in Figur 8 entspricht, gekennzeichnet ist.

4. Kristalline Form von Belumosudilmesylat nach einem der Ansprüche 1 bis 3, die isoliert ist.

5. Kristalline Form nach einem der vorhergehenden Ansprüche, die nicht mehr als etwa 20 %, nicht mehr als etwa 10 %, nicht mehr als etwa 5 %, nicht mehr als etwa 2 %, nicht mehr als etwa 1 % oder etwa 0 % jeglicher anderer Festkörperform von Belumosudilmesylat enthält.

6. Verwendung einer kristallinen Form nach einem der vorhergehenden Ansprüche zur Herstellung einer pharmazeutischen Zusammensetzung.

7. Pharmazeutische Zusammensetzung, umfassend eine kristalline Form nach einem der Ansprüche 1 bis 5 und mindestens einen pharmazeutisch unbedenklichen Hilfsstoff.

8. Verfahren zur Herstellung der pharmazeutischen Zusammensetzung nach Anspruch 7, bei dem man eine kristalline Form nach einem der Ansprüche 1 bis 5 mit mindestens einem pharmazeutisch unbedenklichen Hilfsstoff vereinigt.

9. Kristalline Form nach einem der Ansprüche 1 bis 5 oder pharmazeutische Zusammensetzung nach Anspruch 7 zur Verwendung als Medikament.

10. Kristalline Form nach einem der Ansprüche 1 bis 5 oder pharmazeutische Zusammensetzung nach Anspruch 7 zur Verwendung bei der Behandlung von Graft-Versus-Host-Krankheit einschließlich chronischer Graft-Versus-Host-Krankheit, systemischer Sklerose einschließlich diffuser kutaner systemischer Sklerose, Fibrose einschließlich idiopathischer Lungenfibrose, Plaque-Psoriasis, systemischer Sklerodermie und insbesondere chronischer Graft-Versus-Host-Krankheit und systemischer Sklerose.

11. Verwendung einer kristallinen Form nach einem der Ansprüche 1 bis 5 bei der Herstellung einer anderen Festkörperform von Belumosudil oder einem anderen Salz von Belumosudil oder einer Festkörperform davon.

12. Verfahren zur Herstellung eines Belumosudilsalzes oder einer Festkörperform davon, bei dem man eine kristalline Form nach einem der Ansprüche 1 bis 5 herstellt und sie in eine andere Festkörperform von Belumosudil oder ein anderes Salz oder eine Festkörperform davon umwandelt.

## Revendications

1. Forme cristalline du mésylate de bélumosudil désignée forme M3, qui est **caractérisée par** un diagramme de diffraction des rayons X sur poudre ayant des pics à 7,3, 14,6, 16,6, 17,5 et 19,6 degrés 2-thêta ± 0,2 degré 2-thêta.

2. Forme cristalline de mésylate de bélumosudil selon la revendication 1, qui est **caractérisée par** un diagramme de diffraction des rayons X sur poudre ayant des pics à 7,3, 14,6, 16,6, 17,5 et 19,6 degrés 2-thêta ± 0,2 degré 2-thêta et ayant également un, deux, trois, quatre ou cinq pics supplémentaires choisis parmi 12,9, 13,7, 19,0, 20,6 et 26,0 degrés 2-thêta ± 0,2 degré 2-thêta.

3. Forme cristalline de mésylate de bélumosudil selon la revendication 1 ou la revendication 2, qui est **caractérisée par** un diagramme de diffraction des rayons X sur poudre ayant des pics à 7,3, 12,9, 13,7, 14,6, 16,6, 17,5, 19,0, 19,6, 20,6 et 26,0 degrés 2-thêta ± 0,2 degré 2-thêta, ou un diagramme des rayons X sur poudre essentiellement tel qu'illustré sur la figure 8.

4. Forme cristalline de mésylate de bélumosudil selon l'une quelconque des revendications 1 à 3, qui est isolée.

5. Forme cristalline selon l'une quelconque des revendications précédentes, qui ne contient pas plus d'environ 20 %, pas plus d'environ 10 %, pas plus d'environ 5 %, pas plus d'environ 2 %, pas plus d'environ 1 % ou qui contient environ 0 % d'une quelconque autre forme à l'état solide de mésylate de bélumosudil.

6. Utilisation d'une forme cristalline selon l'une quelconque des revendications précédentes pour la préparation d'une composition pharmaceutique.

7. Composition pharmaceutique comprenant une forme cristalline selon l'une quelconque des revendications 1 à 5, et au moins un excipient pharmaceutiquement acceptable.

8. Procédé pour la préparation d'une composition pharmaceutique selon la revendication 7, comprenant la combinaison d'une forme cristalline selon l'une quelconque des revendications 1 à 5 et d'au moins un excipient pharmaceutiquement acceptable.

9. Forme cristalline selon l'une quelconque des revendications 1 à 5, ou composition pharmaceutique selon la revendication 7, pour une utilisation en tant que médicament.

10. Forme cristalline selon l'une quelconque des revendications 1 à 5, ou composition pharmaceutique selon la revendication 7, pour une utilisation dans le traitement de la maladie du greffon contre l'hôte, y compris la maladie chronique du greffon contre l'hôte, la sclérose systémique, y compris la sclérose systémique cutanée diffuse, la fibrose, y compris la fibrose pulmonaire idiopathique, le psoriasis en plaques, la sclérodermie systémique, et en particulier la maladie chronique du greffon contre l'hôte et la sclérose systémique.

11. Utilisation d'une forme cristalline selon l'une quelconque des revendications 1 à 5, dans la préparation d'une autre forme à l'état solide de bélumosudil, ou d'un autre sel de bélumosudil ou d'une forme à l'état solide de celui-ci.

12. Processus pour la préparation d'un sel de bélumosudil ou d'une forme à l'état solide de celui-ci, comprenant la préparation d'une forme cristalline selon l'une quelconque des revendications 1 à 5, et la conversion de celui-ci en une autre forme à l'état solide de bélumosudil ou d'un autre sel ou d'une forme à l'état solide de celui-ci.
